(19) **Europäisches Patentamt / European Patent Office / Office européen des brevets**

(11) **EP 4 721 704 A1**

(12) **EUROPEAN PATENT APPLICATION**
published in accordance with Art. 153(4) EPC

(43) Date of publication:
08.04.2026   Bulletin 2026/15

(21) Application number: 24815316.5

(22) Date of filing: 22.05.2024

(51) International Patent Classification (IPC):
$A61F\ 2/24^{(2006.01)}$        $A61L\ 27/04^{(2006.01)}$
$A61L\ 27/18^{(2006.01)}$        $A61L\ 27/22^{(2006.01)}$
$A61L\ 27/24^{(2006.01)}$        $A61L\ 27/28^{(2006.01)}$
$A61L\ 27/34^{(2006.01)}$        $A61L\ 27/40^{(2006.01)}$
$A61L\ 27/52^{(2006.01)}$

(52) Cooperative Patent Classification (CPC):
A61F 2/24; A61L 27/04; A61L 27/18; A61L 27/22;
A61L 27/24; A61L 27/28; A61L 27/34; A61L 27/40;
A61L 27/52

(86) International application number:
PCT/JP2024/018763

(87) International publication number:
WO 2024/247832 (05.12.2024 Gazette 2024/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.05.2023   JP 2023088598
03.07.2023   JP 2023109061
09.08.2023   JP 2023129873

(71) Applicant: TEIJIN LIMITED
Kita-ku,
Osaka-shi,
Osaka 530-0005 (JP)

(72) Inventors:
• NAITO, Takaharu
  Osaka-shi, Osaka 530-0005 (JP)
• MOTOYOSHI, Tetsuya
  Osaka-shi, Osaka 530-0005 (JP)
• KITAKAMI, Erika
  Osaka-shi, Osaka 530-0005 (JP)
• KONDO, Takato
  Osaka-shi, Osaka 530-0005 (JP)
• MARUTANI, Koji
  Osaka-shi, Osaka 530-0005 (JP)

(74) Representative: Hoffmann Eitle
Patent- und Rechtsanwälte PartmbB
Arabellastraße 30
81925 München (DE)

(54) **VALVE-FORMING RING, KIT FOR VALVE-FORMING OPERATION, AND METHOD FOR MANUFACTURING VALVE-FORMING RING**

(57)     To provide an annuloplasty ring that can help prevent hemolysis.

An annuloplasty ring comprising (1) a ring-shaped core and (2) a first layer that comprises a fabric or cloth impregnated with a hydrogel, covering the surface of the core, wherein the hydrogel is crosslinked by radiation.

Fig. 1

EP 4 721 704 A1

## Description

FIELD

The present invention relates to an annuloplasty ring, to a valvuloplasty kit and to a method for producing an annuloplasty ring.

BACKGROUND

With an increasingly aging society in recent years, the number of cases of valvular heart disease requiring treatment in the field of cardiovascular surgery has been on an upward trend.

Valvular heart disease is a condition in which a heart valve does not function normally, and specifically, it can be stenosis in which movement of the valve is poor and blood flow is impeded, or regurgitation in which the valve fails to close completely and blood flow is reversed. For mitral valve diseases such as mitral insufficiency or mitral stenosis, or tricuspid valve disease such as tricuspid valve stenosis or tricuspid valve insufficiency, treatment is usually carried out by valve replacement surgery in which the valve itself is replaced with an artificial valve, or by valvuloplasty in which the patient's valve remains but the shape is corrected, with the latter case (valvuloplasty) being the most common approach for treatment. Valvuloplasty has also come to be carried out in cases where valve replacement surgery had previously been used, such as cases with rheumatic changes or cases of infectious endocarditis.

In valvuloplasty, an annuloplasty ring is used to prevent re-dilatation of the annulus after repair of the damaged portions of the valve or chordae tendineae. The annuloplasty ring is generally composed of silicone rubber and a polyethylene terephthalate (PET) fabric present around the periphery, both centered around a metal core of titanium, cobalt alloy or nickel-titanium (see PTL 1 and PTL 2, for example).

[CITATION LIST]

[PATENT LITERATURE]

[PTL 1] Japanese Patent Public Inspection No. 2018-519895
[PTL 2] Japanese Patent Public Inspection HEI No. 4-503318

[NON PATENT LITERATURE]

[NPL 1] Jpn. J. Cardiovasc. Surg. 37:151-154 (2008, Journal of Heart Valve Disease, 2005

SUMMARY

[TECHNICAL PROBLEM]

One problem associated with valvuloplasty is that, following the valvuloplasty procedure, regurgitant jet at the mitral valve impacts with the annuloplasty ring, resulting in hemolysis and requiring reoperation. This is thought to be caused by erythrocytes impacting with the PET fabric, creating shear stress.

In order to solve the problems described above, methods for avoiding hemolysis in a clinical setting are being examined, such as wrapping autologous pericardial tissue around the annuloplasty ring, for example. When such a method is employed, however, the operation time is prolonged due to harvesting of the autologous pericardium, resulting in an increased burden on the patient and physician (NPL 1). It is therefore desirable to develop an annuloplasty ring that can help prevent hemolysis.

It is an object of the present invention to provide an annuloplasty ring that can help prevent hemolysis.

The present invention has been accomplished with the aim of solving the problems described above, and it provides the following aspects.

<First aspect>

[1] An annuloplasty ring comprising (1) a ring-shaped core and (2) a first layer that comprises a fabric or cloth impregnated with a hydrogel, covering the surface of the core, wherein the hydrogel is crosslinked by radiation.

[2] The annuloplasty ring according to [1], wherein (3) a second layer comprising rubber is present between the core and the first layer, and the value of the ratio between the arithmetic mean roughness of the front side of the first layer which is not in contact with the core or the second layer, and the arithmetic mean roughness of the back side of the first layer which is in contact with the core or the second layer, is 0.01 to 0.40.

[3] The annuloplasty ring according to [1] or [2], wherein the first layer comprises an impregnation layer containing a fabric or cloth impregnated with a hydrogel, and a hydrogel layer composed of a hydrogel, the value of the ratio of the stress before subjecting the hydrogel in the hydrogel layer to water absorption treatment and the stress after subjecting the hydrogel to water absorption treatment is 0.05 to 0.80, the water absorption treatment being treatment in which the hydrogel layer is allowed to stand for 30 to 90 minutes in a solution at 30°C to 50°C.

[4] The annuloplasty ring according to any one of [1] to [3], wherein the hydrogel is gelatin or collagen.

[5] The annuloplasty ring according to any one of [1] to [4], wherein the irradiation in radiation crosslinking is at least one type selected from the group consisting of X-ray irradiation, $\gamma$-ray irradiation, electron beam irradiation, UV irradiation and plasma irradiation.

[6] The annuloplasty ring according to any one of [1] to [5], wherein the irradiation in radiation crosslinking is electron beam irradiation.

[7] The annuloplasty ring according to any one of [1] to [6], wherein the fabric or cloth comprises polyester fibers.

[8] The annuloplasty ring according to any one of [1] to [7], wherein the hydrogel is further chemically crosslinked.

[9] An annuloplasty ring comprising (1) a ring-shaped core, and (2) a first layer comprising a fabric or cloth impregnated with a hydrogel, covering the surface of the core.

[10] The annuloplasty ring according to any one of [1] to [9], which is used to help prevent hemolysis in valvuloplasty.

[11] A valvuloplasty kit comprising an annuloplasty ring according to any one of [1] to [10], and a holder and/or sizer.

[12] A method for producing an annuloplasty ring comprising (1) a ring-shaped core and (2) a first layer comprising a fabric or cloth impregnated with a hydrogel, covering the surface of the core, the method comprising a step of irradiating the annuloplasty ring with radiation.

[13] The method according to [12], wherein the step of irradiating with radiation is a step of irradiating with one or more selected from the group consisting of X-rays, $\gamma$-rays, an electron beam, UV and plasma.

[14] The method according to [12] or [13], wherein the step of irradiating with radiation is a step of irradiating with an electron beam.

[15] The method according to any one of [12] to [14], which further comprises a step of adding a crosslinking agent.

<Second aspect>

The present invention also encompasses the following as its second aspect.

[1] An annuloplasty ring comprising (1) a ring-shaped core, and (2) a first layer comprising a fabric or cloth impregnated with a hydrogel, covering the surface of the core.

[2] The annuloplasty ring according to [1], wherein (3) a second layer comprising rubber is present between the core and the first layer.

[3] The annuloplasty ring according to [1], wherein the hydrogel is gelatin or collagen.

[4] The annuloplasty ring according to any one of [1] to [3], wherein the fabric or cloth comprises polyester fibers.

[5] The annuloplasty ring according to any one of [1] to [4], wherein the hydrogel is also chemically crosslinked.

[6] The annuloplasty ring according to any one of [1] to [5], wherein the crosslinking agent in chemical crosslinking is glutaraldehyde or glyoxal.

[7] An annuloplasty ring according to any one of [1] to [6], wherein the value of the ratio between the arithmetic mean roughness of the front side of the first layer which is not in contact with the core or the second layer, and the arithmetic mean roughness of the back side of the first layer which is in contact with the core or the second layer, is 0.01 to 0.40.

[8] The annuloplasty ring according to any one of [1] to [7], wherein the value of the ratio is 0.02 to 0.25.

[9] The annuloplasty ring according to any one of [1] to [8], wherein the value of the ratio is 0.03 to 0.2.

[10] A valvuloplasty kit comprising an annuloplasty ring according to any one of [1] to [9], and a holder and/or sizer.

[11] A method for producing an annuloplasty ring comprising (1) a ring-shaped core and (2) a first layer comprising a fabric or cloth impregnated with a hydrogel, covering the surface of the core, wherein the method comprises the following steps:

(step 1) Step 1 in which a fabric or cloth is provided on the outer side of the ring-shaped core to obtain a composite of a ring-shaped core and a fabric or cloth,

(step 2) Step 2 in which the composite and a hydrogel are contacted to obtain an impregnated composite which is impregnated with the hydrogel,

(step 3) Step 3 in which the impregnated composite is contacted with a crosslinking agent.

[12] The method according to [11], wherein the hydrogel is gelatin or collagen.

[13] The method according to [11] or [12], wherein step 2 is carried out by anchoring the composite onto the central axis of an essentially cylindrical or essentially rectangular columnar container containing a hydrogel.

[14] The method according to any one of [11] to [13], wherein the crosslinking agent is glutaraldehyde or glyoxal.

[15] The method according to any one of [11] to [14], wherein the concentration of the crosslinking agent is 0.01% to 0.7%.

[16] The method according to any one of [11] to [14], wherein the concentration of the crosslinking agent is 0.04% to 0.6%.

The problem relating to the second aspect will now be explained. Specifically, since an annuloplasty ring employs a rigid metal core as a constituent element, this can result in the pressure of the core excessively constraining movement of the valve annulus. This can in turn potentially lead to constriction of the valve or disturbance of blood flow, and avoiding such a situation requires an annuloplasty ring that can follow movement in the direction outside of the plane of the annuloplasty ring (that is, having flexibility in the direction outward from the surface of the annuloplasty ring).

In order to solve this problem there has been proposed an annuloplasty ring that can follow movement of the valve annulus due to modification to the material or cross-sectional shape of the metal core, but such an annuloplasty ring cannot fully follow large degrees of movement when the valve is in a contracted or expanded state, while it also has potentially lower metal fatigue strength with changes in the cross-sectional shape.

It is therefore desirable to develop an annuloplasty ring that can follow movement in the direction outward from its surface. The problem relating to the second aspect is therefore to provide an annuloplasty ring with flexibility in the direction outward from its surface. The effect of the second aspect is, for example, to provide an annuloplasty ring having flexibility in the direction outward from its surface.

<Third aspect>

The present invention also encompasses the following collectively as its third aspect.

[1] An annuloplasty ring comprising (1) a ring-shaped core, and (2) a first layer which comprises an impregnation layer comprising a fabric or cloth impregnated with a hydrogel and a hydrogel layer comprising a hydrogel, covering the surface of the core around its circumference.

[2] The annuloplasty ring according to [1], wherein (3) a second layer comprising rubber is present between the core and the first layer.

[3] The annuloplasty ring according to [1] or [2], wherein the hydrogel is gelatin or collagen.

[4] The annuloplasty ring according to any one of [1] to [3], wherein the fabric or cloth comprises polyester fibers.

[5] The annuloplasty ring according to any one of [1] to [4], wherein the hydrogel is also chemically crosslinked.

[6] The annuloplasty ring according to any one of [1] to [5], wherein the crosslinking agent in chemical crosslinking is glutaraldehyde or glyoxal.

[7] The annuloplasty ring according to any one of [1] to [6], wherein the value of the ratio of the stress before subjecting the hydrogel in the hydrogel layer to water absorption treatment and the stress after subjecting the hydrogel to water absorption treatment is 0.05 to 0.80, where the water absorption treatment is treatment in which the hydrogel layer is allowed to stand for 30 minutes to 90 minutes in a solution at 30°C to 50°C.

[8] The annuloplasty ring according to any one of [1] to [7], wherein the value of the ratio is 0.07 to 0.60.

[9] The annuloplasty ring according to any one of [1] to [8], wherein the value of the ratio is 0.07 to 0.50.

[10] A valvuloplasty kit comprising an annuloplasty ring according to any one of [1] to [9], and a holder and/or sizer.

The problem relating to the third aspect will now be explained. Specifically, an annuloplasty ring is used in valvuloplasty after damaged portions of the valve or chordae tendineae have been repaired. The annuloplasty ring may be composed of, for example, silicone rubber with a polyethylene terephthalate (PET) fabric present on the outermost periphery, centered around a rigid metal core of titanium, cobalt alloy or nickel-titanium (see PTL 1, PTL 2 or USP 6805711), or it may be an artificial valve ring optionally comprising a sheet combining yarn made of a bioabsorbable material with a hydrogel, as one of its constituent elements (Japanese Patent No. 6310167).

The annuloplasty rings described in PTL 1, PTL 2 and USP 6805711 have problems in terms of stability. Specifically, a rigid metal core is used as a constituent element in the annuloplasty ring, but repeated stress causes the stress-concentrated sections to breakdown due to fatigue (i.e. metal fatigue).

Japanese Patent No. 6310167 describes a sheet comprising a combination of yarn made of a bioabsorbable material with a hydrogel, but this publication merely describes potential application in an artificial valve ring. Since the specific construction of the annuloplasty ring is not at all described, none of the aforementioned problems associated with it are addressed. Consequently, the same problems mentioned above will be encountered if the sheet of Japanese Patent No. 6310167 is simply applied as an annuloplasty ring.

It is therefore desirable to develop an annuloplasty ring that can overcome the aforementioned problems of stability, and specifically an annuloplasty ring that can reduce concentration of stress. The problem to be solved by the third aspect is to provide an annuloplasty ring that can reduce concentration of stress (that is, reduce the risk of breakdown due to concentration of stress). One effect of the third aspect is, for example, to provide an annuloplasty ring that can reduce the risk of breakdown caused by concentration of stress.

[ADVANTAGEOUS EFFECTS OF INVENTION]

The annuloplasty ring of the invention can reduce hemolysis.

BRIEF DESCRIPTION OF DRAWINGS

    Fig. 1 is a diagram showing the outer appearance of an annuloplasty ring.
    Fig. 2 is a diagram showing the layered structure of an annuloplasty ring.
    Fig. 3 is a diagram illustrating the flexibility of an annuloplasty ring in the direction outward from the surface and within the surface.
    Fig. 4 is a diagram illustrating in overview an in-plane tensile test for calculation of von Mises stress.
    Fig. 5 is a diagram illustrating the curvature radius of an annuloplasty ring core.

DESCRIPTION OF EMBODIMENTS

<Annuloplasty ring according to first aspect of the invention>

The annuloplasty ring according to the first aspect of the invention comprises (1) a ring-shaped core and (2) a first layer comprising a fabric or cloth impregnated with a hydrogel, covering the surface of the core, wherein the hydrogel is crosslinked by radiation. The annuloplasty ring according to the first aspect of the invention will now be described in detail.

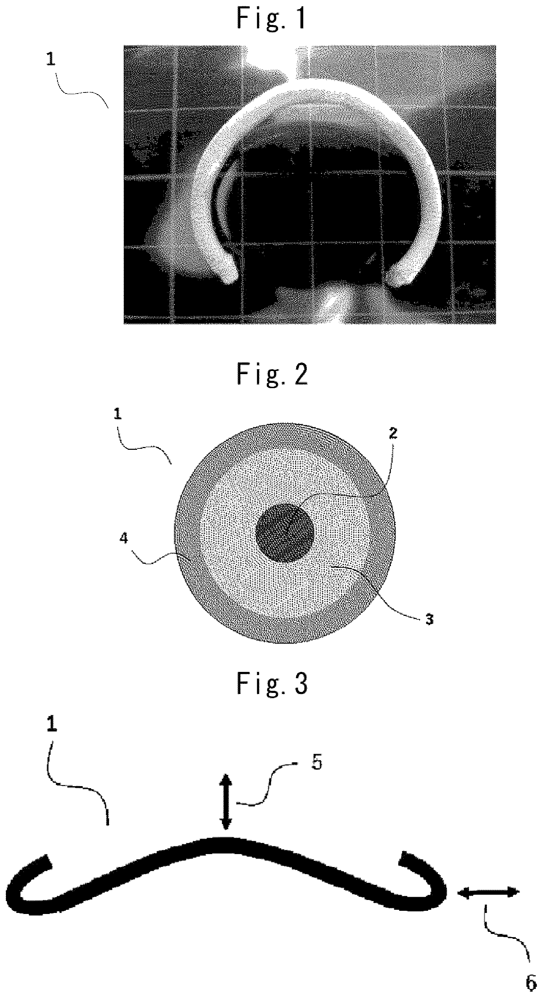

As mentioned above, an annuloplasty ring is used to prevent post-operative annular re-dilatation in valvuloplasty, which is carried out for mitral valve diseases such as mitral insufficiency and mitral stenosis, and for tricuspid valve diseases such as tricuspid valve stenosis and tricuspid valve insufficiency. An annuloplasty ring may also be referred to as a ring for annuloplasty, an artificial valve ring, a valvuloplasty band or an annuloplasty band. The annuloplasty ring of the invention has an outer appearance as shown in Fig. 1, for example. In Fig. 1, 1 corresponds to the annuloplasty ring of the invention. The annuloplasty ring of the invention comprises a core, a first layer and a second layer of the invention, as described below, and these are in the positional relationship shown in Fig. 2. Fig. 2 shows the cross-sectional structure of the annuloplasty ring of the invention, showing the core 2 of the invention, the first layer 4 of the invention and the second layer 3 of the invention.

The annuloplasty ring of the invention is adapted so as to be embedded in the mitral annulus or tricuspid annulus, for example. The annuloplasty ring of the invention therefore preferably has a shape similar to the shape of the mitral annulus or tricuspid annulus. Specifically, the shape may be a complete ring shape surrounding the entire periphery of the valve annulus (a "classic ring"), or it may be a partial ring shape having a notch at part of the valve annulus. Specific examples of such a shape include ellipsoid, C-shaped, D-shaped, kidney-shaped, bean-shaped and egg-shaped forms. The annuloplasty ring as a rigid ring or semi-rigid ring as described below, will usually have a kidney shape or D-shape following the perimeter shape of the mitral annulus (see Japanese Patent Public Inspection No. 2018-519986, for example).

The annuloplasty ring of the invention may also have either bilateral symmetry or left-right asymmetry. The annuloplasty ring of the invention is preferably flat or saddle-shaped. A saddle shape, in particular, reflects the anatomical structure of the natural mitral annulus as opposed to a flat ring, thus allowing a natural valve ring shape to be formed, and dispersing the load on the valve annulus so as to minimize the load transmitted to the leaflet and chordae tendineae. The annuloplasty ring of the invention may be in the form of a flexible ring, rigid ring or semi-rigid ring, with a semi-rigid ring being preferred. This is because if a flexible ring is used it may not be possible to adequately accomplish remodeling of the annulus, while using a rigid ring may not allow proper following of movement of the annulus. The most preferred mode, therefore, is semi-rigid ring that exhibits both flexibility and rigidity.

If the annuloplasty ring of the invention has the shape described above, for example, it will be possible to reduce the anteroposterior diameter of the annulus and increase the bonding area at the front and back tips. This is effective for preventing re-dilatation of the valve and preventing tissue damage at the leaflet joint, while also having an effect of preventing relapse of mitral regurgitation. In addition, since suture reopening that can result in post-operative mitral regurgitation tends to occur near the annulus rather than on the left ventricular side of the leaflet, annuloplasty with an annuloplasty ring also reinforces the suture near the annulus. Moreover, since the mitral annulus becomes rectified into the original normal annulus shape during contraction, an effect of preventing relapse of mitral regurgitation may also be expected as a result of remodeling.

[Core according to first aspect of the invention]

The core according to the first aspect of the invention is a core that determines the shape of the annuloplasty ring of the invention that is able to surround an annulus such as a mitral annulus or tricuspid annulus. The core according to the first aspect of the invention is in a ring-shaped form, and specifically it is in a form corresponding to the shape of the annuloplasty ring of the invention. The cross-sectional shape of the core according to the first aspect of the invention may be circular, rectilinear or a layered structure.

Examples of materials for the core of the first aspect of the invention include metals, and specifically nitinol (also known as nickel-titanium or titanium-nickel alloy), Elgiloy (also known as cobalt-chromium-nickel alloy), alloys composed mainly of nickel, cobalt, chromium and molybdenum, and stainless steel. Preferred among these are nitinol, Elgiloy or stainless steel, which are semi-rigid types with both flexibility and rigidity, as mentioned above. More preferred are superelastic materials such as nitinol or similar superelastic alloys. The term "superelastic" (also referred to as "pseudoelastic") refers to an elastic (reversible) response to applied stress caused by phase transformation between a crystalline austenite phase and a martensite phase. Superelasticity allows materials to be bent beyond the conventional range of specific types of materials such as metal alloys (see USP 11554015).

The core of the first aspect of the invention is obtained using the aforementioned metals as materials, but it may also be partially flexible. For example, adjustment may be made by changing the number of core layers at those portions, by changing the thickness of at least one core cross-section at those portions, by incorporating at least one core material comprising a different material, or by using any combination of these. Adjustment can also be made by inserting a spacer (for example, a polymer spacer and/or elastomer spacer) between at least one pair of adjacent core layers. Another possible embodiment is a braided core knitted with multiple wires, strands and/or braids (see Japanese Patent Public Inspection No. 2018-519986, for example).

The size of the core of the first aspect of the invention may be set as appropriate for the patient in which the annuloplasty ring of the invention is to be applied, and when the cross-section is circular, for example, the diameter may be 300 micrometer to 1500 micrometer, or when the cross-section is rectilinear (such as rectangular), the width may be 300 micrometer to 1500 micrometer and the height may be 300 micrometer to 1500 micrometer, for example. For a multilayer structure, for example, sheet materials each with a width of 300 micrometer to 3000 micrometer and a height of 30 micrometer to 1000 micrometer may be stacked in a group of 2 to 8.

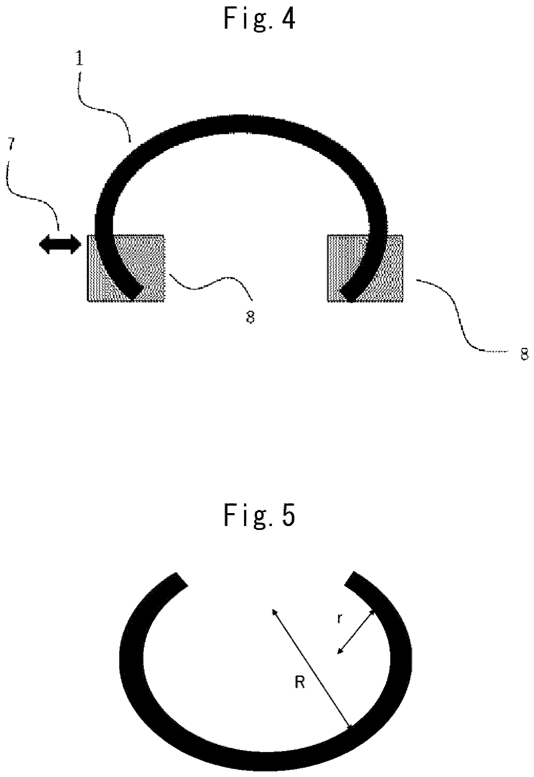

The core of the first aspect of the invention may have a specified curvature radius. Specifically, it may have a first curvature radius R for the core represented as a posterior tip section R and/or an anterior tip section r for the core, as shown in Fig. 5, for example. The value of the curvature radius R may be, as a specific example, 12 to 30, and the value of the curvature radius r may be, as a specific example, 0.5 to 65.

The method of calculating the curvature radius may be a publicly known method employed in the field. For example, the calculation may be based on a method with reference to US Patent Publication 2023/0125047. The terms "posterior tip section" and "anterior tip section" are to be interpreted as is common in the technical field.

[First layer of first aspect of the invention]

The first layer of the first aspect of the invention is one that covers the surface of the core of the first aspect of the invention and comprises a fabric or cloth impregnated with a hydrogel, the hydrogel being crosslinked by radiation.

The fabric or cloth of the first layer of the first aspect of the invention covers the surface of the core of the first aspect of the invention, and it may either cover only part of the core of the first aspect of the invention, or it may cover its entirety. The fabric or cloth is preferably biocompatible, and specifically it may be a biocompatible knitted fabric or woven fabric. A knitted fabric used may have any of various knitted patterns such as a warp knit or weft knit, with particularly preferred examples including double Raschel knitted fabrics, plain stitch fabrics, rib stitch fabrics, tricot fabrics and Raschel fabrics. For example, a warp knitted fabric (wherein the bioabsorbable material has not been decomposed and absorbed) preferably has a density of 20 course/inch to 200 course/inch and 20 wale/inch to 55 wale/inch. More preferably, it is 70 course/inch to 110 course/inch and 30 wale/inch to 45 wale/inch, and even more preferably 80 course/inch to 100 course/inch and 32 wale/inch to 42 wale/inch, from the viewpoint of facilitating suturing of the fabric to biological tissue, and from the viewpoint of water resistance, needle hole leakage and warp knitted fabric anisotropy. The woven fabric used may be a plain weave, twill weave or satin weave.

The fabric or cloth in the first layer of the first aspect of the invention is preferably composed of yarn made of a bioabsorbable material and/or a non-bioabsorbable material.

The yarn composed of the bioabsorbable material is not particularly restricted so long as it is biocompatible and is absorbed by decomposition or disappears in the body with time. Specific examples include polyglycolic acid, polylactic acid, poly(lactide glycolide) copolymer, polyphosphoric acid, polydioxanone, polycaprolactone, polyhydroxyalkanoates, modified polyvinyl alcohol, casein, modified starch, polygalactin-polycaprolactone copolymer, polyglycolic acid-lactic acid copolymer and their derivatives. Preferred among these are one or more selected from the group consisting of polylactic acid, polydioxanone, polyglycolic acid, polygalactin-polycaprolactone copolymer and polyglycolic acid-lactic acid copolymer. This will provide excellent general utility and strength, allowing efficient decomposition and absorption *in vivo.*

The yarn composed of the non-bioabsorbable material is not particularly restricted so long as it is biocompatible and is not absorbed via decomposition and does not disappear in the body with time. Specific examples include fluorine fibers, nylon fibers, polyester fibers such as polyethylene terephthalate, polytrimethylene terephthalate, polybutylene terephthalate, polylactic acid, stereocomplex polylactic acid and polyesters copolymerized with a third component, as well as acrylic fibers, vinylon fibers, vinylidene fibers, polyvinyl chloride fibers, polyethylene fibers, polypropylene fibers, polyurethane fibers, carbon fibers, polystyrene fibers and poly(methyl methacrylate). Preferred among these are one or more selected from the group consisting of fluorine fibers, nylon fibers, polyester fibers and polypropylene fibers. This will provide excellent general utility and strength while maintaining sufficient strength for fabrics and cloths.

The yarn composed of the aforementioned non-bioabsorbable material has a total fineness of preferably 22 dtex to 110 dtex, a single fiber fineness of preferably 0.8 dtex to 5.0 dtex and a filament count of preferably 1 to 48. Adjusting the yarn within this range allows sufficient strength to be maintained while maintaining lightweightness. The total fineness is more preferably 25 dtex to 50 dtex, the single fiber fineness is preferably 1 dtex to 5 dtex and the filament count is preferably 5 to 20.

The yarn composed of the aforementioned bioabsorbable material has a total fineness of preferably 20 dtex to 200 dtex, a single fiber fineness of preferably 1.3 dtex to 44 dtex and a filament count of preferably 1 to 24. Adjusting the yarn within this range allows it to decompose and be absorbed in the body. The total fineness is more preferably 25 dtex to 50 dtex, the single fiber fineness is preferably 1 dtex to 5 dtex and the filament count is preferably 5 to 20.

The yarn composed of the bioabsorbable material and/or the yarn composed of the non-bioabsorbable material may be a monofilament or multifilament. A multifilament yarn can provide a soft hand quality. When multifilaments are used in the body, cellular tissue more easily infiltrates between the monofilaments, which is preferred from the viewpoint of tissue regeneration. However, washing efficiency is higher with monofilament yarn than with multifilaments. In the case of multifilaments, the yarn composed of a bioabsorbable material and/or the yarn composed of a non-bioabsorbable material preferably has a filament count of 5 to 20, and a total fineness of 25 dtex to 50 dtex.

The fabric or cloth of the first layer of the first aspect of the invention is impregnated with a hydrogel. Specific examples of such a hydrogel include gelatin, collagen, polyvinyl alcohol, polyacrylamide, poly-N-vinylpyrrolidone, poly(hydroxylethyl methacrylate), polyethylene oxide, polyethylene glycol, polyethyleneglycol monomethyl ether, cellulose, polyacrylate, polymethacrylate, methyl polyacrylate, polylactic acid, polyvinylsulfonic acid, polyamide, poly(L-lysine), hydrophilic polyurethane, maleic anhydride polymer, protein, fibrin, collagen, cellulosic polymer, methyl cellulose, carboxymethyl cellulose, dextran, carboxymethyldextran, modified dextran, alginates, alginic acid, pectic acid, hyaluronic acid, chitin, pullulan, elastin, laminin, agarose, gellan, chitosan, carboxymethyl starch, chondroitin sulfate, guar, starch, and their copolymers, mixtures and derivatives. Gelatin or collagen is preferred among these, with gelatin being more preferred.

The starting material for gelatin may be a single molecular chain obtained by loosening the triple helix of collagen derived from bovine bone, cowhide, pig skin, bird or fish. The method for producing the gelatin starting material may be an acid treatment method or alkali treatment method (or lime treatment method), and the gelatin material may be produced by any such method or it may be a commercially available gelatin starting material. Since commercially available gelatin starting materials are subjected to various refining steps during the production process up until their extraction, they have few components other than proteins, and generally have a composition with a protein content of 85% or greater, a water content of 8 to 14%, an ash content of 2% or less, and no greater than 1% of other components (such as lipids or polysaccharides), and such common gelatin starting materials may also be used for the invention.

Since the gelatin starting material is heat-denatured collagen its amino acid composition is approximately the same as collagen, but it has a very specific composition compared to other proteins, with glycine constituting about 1/3 of the total, and repeating once for every 3 amino acids in the sequence. The method of discriminating collagen and gelatin may be, for example, observation of the signal at 31.5° obtained by wide-angle X-ray measurement. The signal at 31.5° is a signal derived from the triple helix structure of collagen, and when the triple helix structure characteristic of collagen loosens to form gelatin, the signal disappears.

The content of the gelatin starting material in the gelatin solution is preferably 1 wt% to 70 wt% and more preferably 3 wt% to 20 wt%.

The solvent used to dissolve the gelatin starting material is water, but (polyhydric) alcohols or dimethyl sulfoxide may also be added as necessary for viscosity adjustment.

The method of impregnating the hydrogel into the fabric or cloth of the first layer of the first aspect of the invention may be a method commonly employed in the relevant field, and a specific example is a method of spraying or coating the fabric or cloth with the hydrogel. More specifically, it is carried out by a method in which the fabric or cloth is impregnated with a solution containing a hydrogel (for example, beMatrix gelatin LS-H by Nitta Gelatin Inc.), and cooling or drying is repeated at a specified temperature". The temperature of the hydrogel solution for this method may be 20°C to 80°C, for example, and the impregnating time may be several seconds to several minutes, for example. The time for cooling or drying after impregnation may be 2 minutes to 24 hours, for example.

When the hydrogel is a protein, for example, the coverage of the hydrogel on the fabric or cloth (the solid mass per area) will usually be 1 mg/cm$^2$ to 200 mg/cm$^2$, preferably 1 mg/cm$^2$ to 100 mg/cm$^2$, more preferably 2 mg/cm$^2$ to 80 mg/cm$^2$ and even more preferably 2 to 60 mg/cm$^2$.

The fabric or cloth of the first layer of the first aspect of the invention has the hydrogel crosslinked by radiation. The phrase "hydrogel crosslinked by radiation" means that the hydrogel is crosslinked by irradiation in the manner described below.

Examples for the irradiation for radiation crosslinking include X-ray irradiation, $\gamma$-ray irradiation, electron beam irradiation, UV irradiation and plasma irradiation. Electron beam irradiation is preferred among these from the viewpoint of the hemolysis rate. The irradiation for the radiation crosslinking may be of only one type of radiation, or several different types of radiation may be used in combination. For a combination of multiple different types, the first irradiation may be, for example, irradiation with radiation in a manner to obtain a low crosslinking degree (for example, UV irradiation), followed by second irradiation as radiation to obtain a high crosslinking degree (for example, electron beam irradiation). This will allow a higher-strength annuloplasty ring to be obtained.

The irradiation conditions for the radiation crosslinking are preferably an acceleration voltage of 10 kilovolt to 8000 kilovolt, and an exposure dose of 5 kilogray to 200 kilogray, more preferably 10 kilogray to 60 kilogray and even more preferably 20 to 40 kilogray. The exposure time for the radiation may be adjusted as appropriate. The temperature for the radiation exposure will usually be minus 80°C to 40°C, with adjustment as appropriate. The irradiation may also be carried out in the presence of a desiccant or deoxidizer, which can inhibit ozone generation while also inhibiting inactivation of the radicals produced by electron beam irradiation, by oxygen in the atmosphere.

The specific method of radiation exposure may be a method commonly employed in the field (see Japanese Unexamined Patent Publication No. 2020-157516 and Japanese Unexamined Patent Publication No. 2021-146242, for example), and as an example, the first layer according to the first aspect of the invention which has been impregnated with the hydrogel may be irradiated under the following conditions. However, the following is merely an example and is not intended to be limitative.

Irradiation device: Electron beam irradiation device (for example, Dynamitron electron accelerator by RDI)
Dosimeter: CTA dosimeter device (for example, UV1800 spectrophotometer by Shimadzu Corp.)
Dose meter: CTA dose meter (for example, FTR-125 by FujiFilm Corp.)
Acceleration voltage: 4.8 megavolt
Current: 20 milliampere
Table speed (outer periphery): 18.0 m/min
Exposure dose: 30 kilogray
Irradiation direction: One side
Irradiations: 2
Support material: cardboard stack

The crosslinking degree (decomposition degree and decomposition rate) of the radiation crosslinked hydrogel described above can be evaluated using the absorbance before and after irradiation as the index. The method is based on the phenomenon that progressive decomposition of the hydrogel increases absorbance, and it is therefore commonly employed in the field. Specifically, the absorbance at the time of complete decomposition of the hydrogel and the absorbance at a certain time point thereafter are measured, and the ratio is calculated as the decomposability of the hydrogel. Based on this, the decomposition rate of the hydrogel before irradiation and the decomposition rate of the hydrogel after irradiation are each calculated, and their ratio (hydrogel decomposition rate after irradiation/hydrogel decomposition rate before irradiation) is calculated.

A radiation-crosslinked hydrogel is used as one of the constituent elements in the annuloplasty ring of the first aspect of the invention, as mentioned above, and surprisingly, it has been found that employing such a construction makes it possible to inhibit hemolysis. This is also clear from the description in the Examples below.

The fabric or cloth of the first layer of the first aspect of the invention may also have the hydrogel chemically crosslinked. The phrase "hydrogel chemically crosslinked" means that the hydrogel is crosslinked by a crosslinking agent in the manner described below.

Examples of crosslinking agents for chemical crosslinking include aldehyde-based crosslinking agents (such as glutaraldehyde, formaldehyde and glyoxal), isocyanate-based crosslinking agents (such as hexamethylene diisocyanate), carbodido-based crosslinking agents (1-ethyl-3-(3-dimethylaminopropyl)carbodiimide hydrochloride), polyepoxy-based crosslinking agents (such as ethyleneglycol diethyl ether) and transglutaminases, with aldehyde-based crosslinking agents being preferred, glutaraldehyde and glyoxal being more preferred, and glutaraldehyde being especially preferred.

The method of chemically crosslinking the hydrogel in the fabric or cloth of the first layer of the first aspect of the invention may be a method commonly employed in the field, and for example, the first layer of the first aspect of the invention impregnated with a hydrogel may be impregnated with a solution containing a crosslinking agent (for example, 50% glutaraldehyde solution by Tokyo Chemical Industry Co., Ltd.). The reaction time for impregnation of the solution containing the crosslinking agent will usually be 30 minutes to 24 hours, with adjustment as appropriate. The temperature for the impregnation will usually be 0°C to 50°C, with adjustment as appropriate. The concentration of the crosslinking agent for the chemical crosslinking will usually be 0.01 mass% to 10 mass%.

The thickness of the first layer of the first aspect of the invention may be appropriately set depending on the patient to whom the annuloplasty ring of the invention is to be applied, and for example, it may be 100 micrometers to 2000 micrometers, preferably 150 micrometers to 1700 micrometers and more preferably 200 micrometers to 1500 micrometers.

The annuloplasty ring of the invention may also comprise a rubber-containing second layer (hereunder also abbreviated as "second layer of the first aspect of the invention"), in addition to the core and first layer of the first aspect of the invention. The second layer of the first aspect of the invention comprises rubber as described below, and its positional relationship is between the core of the first aspect of the invention and the first layer of the first aspect of the invention.

The rubber in the second layer of the first aspect of the invention may be synthetic rubber such as silicone rubber, latex rubber, styrene-butadiene-styrene (SBS), styrene-isoprene-styrene (SIS), styrene-ethylene-butylene-styrene (SEBS) or styrene-ethylene-propylene-styrene (SEPS), or fluorinated silicone rubber, fluorine rubber, chloroprene rubber, nitrile rubber, acrylic rubber, epichlorohydrin rubber, isoprene rubber, styrene-butadiene rubber, butadiene rubber, ethylene-propylene rubber, chlorinated polyethylene rubber, chlorosulfonated polyethylene rubber or butyl rubber. Silicone rubber is preferred among these. The rubber may be one that can be sewed with sewing thread.

The thickness of the second layer of the first aspect of the invention may be appropriately set depending on the patient to whom the annuloplasty ring of the first aspect of the invention is to be applied, and it may be 50 micrometer to 1500 micrometers, for example.

The annuloplasty ring of the first aspect of the invention may also be one wherein (3) a second layer comprising rubber is present between the core and the first layer, and the value of the ratio between the arithmetic mean roughness of the front side of the first layer which is not in contact with the core or the second layer, and the arithmetic mean roughness of the back side of the first layer which is in contact with the core or the second layer, is 0.01 to 0.40. Specific examples and preferred examples of this aspect will be understood by referring to the annuloplasty ring of the second aspect of the invention as described below.

The annuloplasty ring of the first aspect of the invention may also be one wherein the first layer comprises an impregnation layer comprising a fabric or cloth impregnated with a hydrogel, and a hydrogel layer composed of a hydrogel, the value of the ratio of the stress before subjecting the hydrogel in the hydrogel layer to water absorption treatment and the stress after subjecting the hydrogel to water absorption treatment being 0.05 to 0.80, and the water absorption treatment being treatment in which the hydrogel layer is allowed to stand for 30 to 90 minutes in a solution at 30°C to 50°C. Specific examples and preferred examples of this aspect will be understood by referring to the annuloplasty ring of the third aspect of the invention as described below.

<Method for producing annuloplasty ring according to first aspect of the invention>

The method for producing an annuloplasty ring according to the invention (hereunder also abbreviated as "production method of the first aspect of the invention") is a method for producing an annuloplasty ring comprising (1) the aforementioned ring-shaped core and (2) a first layer comprising a fabric or cloth impregnated with a hydrogel, covering the surface of the core, the method comprising a step of irradiating the annuloplasty ring with radiation (hereunder also abbreviated as "irradiation step of the invention").

The annuloplasty ring to be produced by the production method of the invention is as described above.

More specifically, the production method of the invention includes a step of preparing a core of the first aspect of the invention (hereunder also abbreviated as "core preparation step of the invention"), a step of preparing a first layer of the first aspect of the invention (hereunder also abbreviated as "first layer preparation step of the invention"), and an irradiation step of the invention.

The core preparation step of the invention is a step of preparing a core of the first aspect of the invention, described above. Specifically, for example, a commercially available stainless steel ring (such as a stainless steel ring by Maruho Hatsujo Kogyo) may be prepared and adjusted to the desired size as necessary.

The actual core of the first aspect of the invention is the same as explained above for the annuloplasty ring of the first aspect of the invention, and the specific and preferred examples thereof are also the same.

The first layer preparation step of the invention is a step of preparing a first layer of the first aspect of the invention, as described above. A specific example is a method of preparing a fabric or cloth impregnated with a hydrogel, and more specifically, the first layer of the first aspect of the invention may be wrapped around a core of the first aspect of the invention or a second layer of the first aspect of the invention, while being sewed or directly covered. The method of sewing may be any one commonly employed in the field, and for example, the sewing may be carried out by hand sewing or machine (e.g., a sewing machine). The actual first layer of the first aspect of the invention is the same as explained above for the annuloplasty ring of the first aspect of the invention, and the specific and preferred examples thereof are also the same.

The irradiation step of the invention is a step of irradiating an annuloplasty ring comprising a core of the first aspect of the invention and a first layer of the first aspect of the invention, obtained by a core preparation step and first layer preparation step according to the invention. By carrying out this step, the hydrogel in the annuloplasty ring of the first aspect of the invention is crosslinked by radiation. The type and method of irradiation are the same as explained above for the annuloplasty ring of the first aspect of the invention, and the specific and preferred examples thereof are also the same.

The production method of the invention may further include a step of adding a crosslinking agent to an annuloplasty ring that comprises a core of the first aspect of the invention and a first layer of the first aspect of the invention, obtained by the core preparation step and first layer preparation step of the invention (this step may also be referred to herein simply as "crosslinking agent addition step of the invention"). By carrying out this step, the hydrogel in the annuloplasty ring of the invention becomes chemically crosslinked. The type of crosslinking agent and the crosslinking method are the same as explained above for the annuloplasty ring of the first aspect of the invention, and the specific and preferred examples thereof are also the same.

The crosslinking agent addition step of the invention is preferably carried out (1) after the first layer preparation step of the invention and before the irradiation step of the invention, or (2) after the irradiation step of the invention.

The production method of the invention may further include a step of preparing the second layer of the first aspect of the invention (also abbreviated herein as "second layer preparation step of the invention"). The second layer preparation step of the invention is a step of preparing a second layer of the first aspect of the invention, and as a specific example, it may be a method of preparing a rubber-containing second layer which is present between a core of the first aspect of the invention and a first layer of the first aspect of the invention. Specifically, for example, it may be a method in which a second layer of the first aspect of the invention covers a core of the first aspect of the invention and both ends are sealed with liquid silicone, for example, or a method in which a die that is larger than the core of the first aspect of the invention is prepared, and the core and second layer of the first aspect of the invention are inserted therein and integrally molded. The actual second layer of the first aspect of the invention is the same as explained above for the annuloplasty ring of the first aspect of the invention, and the specific and preferred examples thereof are also the same.

The second layer preparation step of the invention is preferably carried out after the core preparation step of the invention and before the first layer preparation step of the invention.

<Valvuloplasty kit according to first aspect of the invention>

The valvuloplasty kit of the first aspect of the invention (hereunder also abbreviated as "kit of the first aspect of the invention") comprises an annuloplasty ring of the first aspect of the invention, and a holder and/or sizer. The annuloplasty ring of the first aspect of the invention is as explained for the annuloplasty ring of the first aspect of the invention described above. The holder in the kit of the first aspect of the invention houses the annuloplasty ring of the first aspect of the invention. By using the holder it is possible for a health care professional such as a physician to move the annuloplasty ring of the first aspect of the invention to a specified site in the patient. A sizer in the kit of the first aspect of the invention is a surgical instrument used for measurement of the opening in which the annuloplasty ring is to be planted, and it is guided by hand and used during valvuloplasty.

The kit of the first aspect of the invention may also include an instruction sheet if necessary. The instruction sheet may contain essential text and drawings describing the principle, features and operating procedure for the annuloplasty ring of the first aspect of the invention, and may be in the form of an instruction manual, package insert pamphlet or leaflet.

The kit of the first aspect of the invention is used for mitral valve disease such as mitral insufficiency or mitral stenosis, and for valvuloplasty for tricuspid valve disease such as tricuspid valve stenosis or tricuspid valve insufficiency. By using a kit of the first aspect of the invention it is possible to perform valvuloplasty in a more convenient, rapid and accurate manner.

<Annuloplasty ring of second aspect of the invention>

The annuloplasty ring of the second aspect of the invention comprises (1) a ring-shaped core and (2) a first layer comprising a fabric or cloth impregnated with a hydrogel, covering the surface of the core. The annuloplasty ring of the second aspect of the invention will now be described in detail.

[Core according to second aspect of the invention]

Specific and preferred examples for the core of the second aspect of the invention will be understood by referring to the core of the first aspect of the invention as described above.

[First layer of second aspect of the invention]

Specific and preferred examples for the first layer of the second aspect of the invention will be understood by referring to the first layer of the first aspect of the invention as described above.

[Second layer of second aspect of the invention]

Specific and preferred examples for the second layer of the second aspect of the invention will be understood by referring to the second layer of the first aspect of the invention as described above.

[Arithmetic mean roughness according to second aspect of the invention]

The annuloplasty ring of the second aspect of the invention has the structure described above, and the value of the ratio between the arithmetic mean roughness on the front side of the first layer of the second aspect of the invention, which is not in contact with the core or second layer of the second aspect of the invention, and the arithmetic mean roughness on the back side of the first layer of the second aspect of the invention, which is in contact with the core or second layer of the second aspect of the invention, is 0.01 to 0.40, for example, and preferably 0.02 to 0.25, more preferably 0.03 to 0.2 and most preferably 0.04 to 0.15.

The arithmetic mean roughness is the mean value of the distance from a reference line representing the average value of irregularities on the surface. Thus, a smaller value for the arithmetic mean roughness may be considered to correspond to a smoother plane with fewer irregularities.

As demonstrated by the Examples described below, the annuloplasty ring of the second aspect of the invention has been found, surprisingly, to have increased flexibility in the direction outward from the surface of the annuloplasty ring, if the arithmetic mean roughness is as specified above. The annuloplasty ring of the second aspect of the invention therefore preferably has the arithmetic mean roughness ratio as specified above.

The flexibility in the direction outward from the surface is the flexibility (degree of widening) of the annuloplasty ring in the Z-axial direction, as illustrated in Fig. 3. Fig. 3 shows the flexibility of an annuloplasty ring 1, where "flexibility in the direction outward from the surface" means the flexibility (degree of widening) in the direction of the arrow 5. On the other hand, "flexibility in the direction within the surface", which is the opposite of the aforementioned "flexibility in the direction outward from the surface" means flexibility (degree of widening) in the direction of the arrow 6.

The method of measuring the arithmetic mean roughness may be a method commonly employed in the relevant field. For example, the annuloplasty ring of the second aspect of the invention may be anchored on a surface plate parallel to the ground and measured using a measuring device (for example, a One-Shot 3D Measuring Macroscope (VR-6000 by Keyence Corp.)) under the following conditions. The following conditions are only for illustration and are not intended to be limitative. The location where the arithmetic mean roughness is measured may be the entire annuloplasty ring, or it may be a portion (for example, an arbitrary region cut out to about 4 mm; such as a region at either of both end portions or the center portion where the ring-shaped annuloplasty ring is a straight line). Other detailed measuring methods may be carried out with reference to the description in the Examples below.

· Roughness standard: JIS B 0601:1994, 2001
· Cutoff value: 0.8 mm
· Reference value: 4 mm

The method of calculating the arithmetic mean roughness ratio is carried out using the following formula.

Arithmetic mean roughness ratio = arithmetic mean roughness of front side of first layer of second aspect of the invention/arithmetic mean roughness of back side of first layer of second aspect of the invention <Formula 1>

The construction, materials and properties of the annuloplasty ring of the second aspect of the invention may be as appropriate, with reference to the aspects described for the annuloplasty ring of the first aspect of the invention.

<Method for producing annuloplasty ring of second aspect of the invention>

The method for producing an annuloplasty ring according to a second aspect of the invention (hereunder also abbreviated as "production method of second aspect of the invention") is a method for producing an annuloplasty ring comprising (1) the aforementioned ring-shaped core and (2) a first layer comprising a fabric or cloth impregnated with a hydrogel, covering the surface of the core, the method comprising step 1 in which a fabric or cloth is provided on the outer side of the ring-shaped core to obtain a composite of the ring-shaped core and the fabric or cloth (hereunder also referred to as "step 1 of the second aspect of the invention"), step 2 in which the composite is contacted with the hydrogel to obtain an impregnated composite that has been impregnated with the hydrogel (hereunder also referred to as "step 2 of the second aspect of the invention"), and step 3 in which the impregnated composite is contacted with a crosslinking agent (hereunder also abbreviated as "step 3 of the second aspect of the invention").

Needless to mention, the annuloplasty ring to be produced by the production method of the second aspect of the invention is the same as described above. Therefore the aforementioned description relating to "annuloplasty ring of the second aspect of the invention" (or "annuloplasty ring of the first aspect of the invention", as necessary) may be applied.

[Step 1 of second aspect of the invention]

Step 1 of the second aspect of the invention is a step in which a fabric or cloth is provided on the outer side of a ring-shaped core to obtain a composite of the ring-shaped core and the fabric or cloth.

Step 1 of the second aspect of the invention is carried out by first preparing the aforementioned core of the second aspect of the invention. Specifically, for example, a commercially available stainless steel ring (such as a stainless steel ring by Maruho Hatsujo Kogyo) may be prepared and adjusted to the desired size as necessary.

The actual core of the second aspect of the invention is the same as explained above for the annuloplasty ring of the second aspect of the invention, and the specific and preferred examples thereof are also the same.

Step 1 of the second aspect of the invention is followed by providing a fabric or cloth on the outer side (surface) of the core of the second aspect of the invention. Specifically, the fabric or cloth is wrapped around a core of the second aspect of the invention, while being sewn to or directly covering it. The method of sewing may be any one commonly employed in the field, and for example, the sewing may be accomplished by hand sewing or machine (e.g., a sewing machine).

When a second layer according to the second aspect of the invention is provided in the annuloplasty ring of the second aspect of the invention, step 1 is carried out by providing a second layer according to a second aspect of the invention on the outer side (surface) of the core of the second aspect of the invention, and further providing a fabric or cloth on the outer side (surface). In this case, the method may be, as a specific example, a method in which the second layer of the second aspect of the invention covers a core of the second aspect of the invention and both ends are sealed with liquid silicone, for example, or a method in which a die that is larger than the core of the second aspect of the invention is prepared, and the core and second layer of the second aspect of the invention are inserted therein and integrally molded.

The actual second layer of the second aspect of the invention is as explained above for the annuloplasty ring of the second aspect of the invention (including the annuloplasty ring of the first aspect of the invention as necessary), and the specific examples and preferred examples thereof are also the same.

Step 1 of the second aspect of the invention can produce a composite of a ring-shaped core with a fabric or cloth.

[Step 2 of second aspect of the invention]

Step 2 of the second aspect of the invention is a step in which a composite obtained by step 1 of the second aspect of the invention is contacted with a hydrogel to obtain a covered composite impregnated with the hydrogel.

The method of contacting the composite with the hydrogel may be a method commonly employed in the relevant field, a specific example of which is the method described above for "annuloplasty ring of second aspect of the invention" (method of spraying or coating hydrogel).

In step 2 of the second aspect of the invention, a method using a die may be employed in addition to or instead of the method described above. A method using a die may be, specifically, a method of anchoring the composite onto the central axis of the hydrogel-containing container. This method allows the hydrogel to be uniformly impregnated into the composite. Specifically, the shape of the container may be cylindrical or polygonal columnar such as triangular columnar, square columnar or pentagonal columnar. In a method using such a die, the time required to fix the hydrogel is from several seconds to 24 hours, with a fixing temperature of from 0°C to room temperature, and with the fixing being optionally followed by a time period for drying as necessary.

The actual hydrogel for step 2 of the second aspect of the invention is as explained above for the annuloplasty ring of the second aspect of the invention (including the annuloplasty ring of the first aspect of the invention as necessary), and the specific and preferred examples thereof are also the same.

Step 2 of the second aspect of the invention described above can produce an impregnated composite that is impregnated with a hydrogel.

[Step 3 of the second aspect of the invention]

Step 3 of the second aspect of the invention is a step in which the impregnated composite obtained by step 2 of the second aspect of the invention is contacted with a crosslinking agent. By carrying out step 3 of the second aspect of the invention, the hydrogel itself in the annuloplasty ring of the second aspect of the invention becomes chemically crosslinked. The type of crosslinking agent and the crosslinking method are as explained above for the annuloplasty ring of the second aspect of the invention (including the annuloplasty ring of the first aspect of the invention as necessary), and the specific examples and preferred examples thereof are also the same.

By carrying out steps 1 to 3 of the second aspect of the invention, it is possible to produce an annuloplasty ring comprising (1) a ring-shaped core and (2) a first layer comprising a fabric or cloth impregnated with a hydrogel, covering the surface of the core.

<Valvuloplasty kit according to second aspect of the invention>

Specific examples and preferred examples for the valvuloplasty kit of the second aspect of the invention will be understood by referring to the valvuloplasty kit of the first aspect of the invention described above.

<Annuloplasty ring of third aspect of the invention>

The annuloplasty ring of the third aspect of the invention comprises (1) a ring-shaped core, and (2) a first layer which comprises an impregnation layer comprising a fabric or cloth impregnated with a hydrogel, and a hydrogel layer comprising a hydrogel, covering the surface of the core around its circumference. The annuloplasty ring of the third aspect of the invention will now be described in detail.

[Core according to third aspect of the invention]

Specific and preferred examples for the core of the third aspect of the invention will be understood by referring to the core of the first aspect of the invention as described above.

[First layer of third aspect of the invention]

The first layer of the third aspect of the invention covers the surface of the core of the third aspect of the invention around its circumference, and it includes a first layer which comprises an impregnation layer comprising a fabric or cloth impregnated with a hydrogel, and a hydrogel layer composed of a hydrogel. In the annuloplasty ring of the third aspect of the invention, since the first layer of the third aspect of the invention having the specified structure thus covers the surface of the core of the third aspect of the invention around its circumference, for example, it is possible to inhibit concentration of stress as demonstrated in the Examples described below, and as a result to overcome the problem of stability caused by fatigue fracture.

The term "circumference" referred to here means the ring shape of the core of the third aspect of the invention, and "around its circumference" is synonymous with "covering over the entire ring (form) of the core".

The fabric or cloth of the first layer of the third aspect of the invention is preferably biocompatible, and specifically it may be a biocompatible knitted fabric or woven fabric. A knitted fabric used may have any of various knitted patterns such as a warp knit or weft knit, with particularly preferred examples including double Raschel knitted fabrics, plain stitch fabrics, rib stitch fabrics, tricot fabrics and Raschel fabrics. For example, a warp knitted fabric (wherein the bioabsorbable material has not been decomposed and absorbed) preferably has a density of 20 course/inch to 200 course/inch and 20 wale/inch to 55 wale/inch. More preferably, it is 70 course/inch to 110 course/inch and 30 wale/inch to 45 wale/inch, and even more preferably 80 course/inch to 100 course/inch and 32 wale/inch to 42 wale/inch, from the viewpoint of facilitating suturing of the fabric to biological tissue, and from the viewpoint of water resistance, needle hole leakage and warp knitted fabric anisotropy. The woven fabric used may be a plain weave, twill weave or satin weave.

The fabric or cloth in the first layer of the third aspect of the invention is preferably composed of yarn made of a bioabsorbable material and/or a non-bioabsorbable material.

The yarn composed of a bioabsorbable material is not particularly restricted so long as it is biocompatible and is absorbed by decomposition or disappears in the body with time. Specifically, it may be one as described for the first layer of the first aspect of the invention.

The yarn composed of a non-bioabsorbable material is also not particularly restricted so long as it is biocompatible and is not absorbed by decomposition and does not disappear in the body with time. Specifically, it may be one as described for the first layer of the first aspect of the invention.

The first layer of the third aspect of the invention comprises an impregnation layer which comprises a fabric or cloth and is impregnated with a hydrogel, which means that the fabric or cloth is also impregnated with the hydrogel. The term "impregnated" used here means, for example, a form in which the hydrogel is infiltrating into the fabric or cloth, but the hydrogel may simply cover (envelop) the fabric or cloth.

Specific examples of such hydrogels include those mentioned for the first layer of the first aspect of the invention.

The first layer of the third aspect of the invention comprises a hydrogel layer comprising a hydrogel.

The hydrogel in the hydrogel layer of the first layer of the third aspect of the invention is in the layer comprising the hydrogel that has not been impregnated into the fabric or cloth when the fabric or cloth has been contacted with the hydrogel, as explained above for the impregnation layer of the first layer of the third aspect of the invention, and it may also be referred to as a layer composed of a hydrogel alone, or a layer composed essentially of a hydrogel alone. The hydrogel in the hydrogel layer is as explained above for the impregnation layer, and the specific and preferred examples thereof are also the same.

The structures and positional relationship between the impregnation layer and the hydrogel layer in the first layer of third aspect of the invention may have any of the following patterns, for example. Specifically, the patterns include (pattern 1): a pattern wherein the impregnation layer covers the surface of the core of the third aspect of the invention, and its surface is further covered by the hydrogel layer, (pattern 2): a pattern wherein the surface of the core of the third aspect of the invention is covered with the hydrogel layer, and its surface is further covered with the impregnation layer, (pattern 3): for cases where the fabric or cloth and the hydrogel are in a simple covering relationship in the impregnation layer, a pattern wherein the surface of the core of the third aspect of the invention is covered with the hydrogel layer, its surface is further covered by the fabric or cloth, and that surface is still further covered with the hydrogel, and (pattern 4): for cases where the fabric or cloth and the hydrogel are in a simple covering relationship in the impregnation layer, a pattern wherein the surface of the core of the third aspect of the invention is covered by the fabric or cloth, and its surface is further covered by the hydrogel (including the hydrogel layer).

The thickness of the first layer of the third aspect of the invention may be appropriately set depending on the patient to whom the annuloplasty ring of the third aspect of the invention is to be applied, and for example, it may be 100 micrometers to 2000 micrometers, preferably 130 micrometers to 1000 micrometers, more preferably 150 micrometers to 800 micrometers, even more preferably 170 micrometers to 600 micrometers and most preferably 200 to 450 micrometers.

[Second layer of third aspect of the invention]

The annuloplasty ring of the third aspect of the invention may also comprise a rubber-containing second layer (hereunder also referred to as "second layer of the third aspect of the invention"), in addition to the core and first layer of the third aspect of the invention. The second layer of the third aspect of the invention comprises rubber as described below, and its positional relationship is between the core of the third aspect of the invention and the first layer of the third aspect of the invention. Specific and preferred examples for the second layer of the third aspect of the invention will be understood by referring to the second layer of the first aspect of the invention.

[Stress of hydrogel according to third aspect of the invention]

The annuloplasty ring of the third aspect of the invention has the structure described above, and the ratio of the stress before application of the hydrogel in the hydrogel layer for water absorption treatment and the stress after application of the hydrogel for water absorption treatment, is 0.05 to 0.80, for example, preferably 0.07 to 0.60, more preferably 0.07 to 0.50 and most preferably 0.07 to 0.46. According to another aspect, the value of the ratio may be 0.07 ($\pm$0.02) or 0.4 ($\pm$0.02).

Water absorption treatment is treatment in which the hydrogel layer is allowed to stand for 30 to 90 minutes in a solution at 30°C to 50°C. The temperature is preferably 37°C, the solution is preferably water, and the standing time is preferably 60 minutes. Specific examples for water include ultrapure water, distilled water, deionized water and purified water, with ultrapure water being preferred.

The stress referred to above is stress produced per unit area ($mm^2$) ($N/mm^2$), and it may also be referred to as "degree of stress". The stress may be measured, for example, by measuring the thickness (film thickness) of a hydrogel sample and using a prescribed tester (for example, EZ-SX by Shimadzu Corp.). Specifically, a tensile jig (for example, a 500 N screw-type flat grip by Shimadzu Corp.) is used to sandwich the sample from above and below at a location 8.5 mm from the edge, and then the sample is displaced until it fractures, recording the stress upon fracture (the speed being 200 mm/min, for example). Other detailed measuring methods may be carried out with reference to the description in the Examples below.

As demonstrated by the examples described below, the annuloplasty ring of the third aspect of the invention has been found, surprisingly, to remarkably inhibit concentration of stress, and as a result to overcome the problem of stability due to fatigue fracture, if the aforementioned ratio is as specified above. The present inventors found that when the aforementioned crosslinking degree of the hydrogel is low, the hydrogel tends to be more easily broken down by water absorption, and this knowledge was used to set the aforementioned parameter (the ratio). The present inventors have been the first to demonstrate the relationship between this parameter and reduction of stress concentration.

The annuloplasty ring of the third aspect of the invention therefore preferably has the ratio value specified above.

The method of calculating the ratio value is carried out using the following formula.

<Formula 2>

Ratio = Stress after water absorption treatment/stress before water absorption treatment

The construction, materials and properties of the annuloplasty ring of the third aspect of the invention may be as appropriate, with reference to the aspects described for the annuloplasty ring of the first aspect of the invention.

<Method for producing annuloplasty ring of third aspect of the invention>

The method for producing an annuloplasty ring of the third aspect of the invention (hereunder also abbreviated as "production method according to the third aspect of the invention") is a method for producing an annuloplasty ring comprising (1) the aforementioned ring-shaped core, and (2) a first layer which comprises an impregnation layer comprising a fabric or cloth impregnated with a hydrogel and a hydrogel layer comprising a hydrogel, covering the surface of the core around its circumference, wherein the method comprises the following steps: step 1 in which the fabric or cloth is provided on the outer side of the ring-shaped core to obtain a composite of the ring-shaped core with the fabric or cloth (hereunder also referred to as "step 1 of the third aspect of the invention"), and step 2 in which the composite is contacted with the hydrogel to obtain a first layer which comprises an impregnation layer comprising the fabric or cloth impregnated with the hydrogel, and a hydrogel layer comprising a hydrogel (hereunder also referred to as "step 2 of the third aspect of the invention").

Needless to mention, the annuloplasty ring to be produced by the production method of the invention is the same as described above. Thus, the aspects explained for the annuloplasty ring of the third aspect of the invention therefore apply as appropriate.

[Step 1 of third aspect of the invention]

Step 1 of the third aspect of the invention is a step in which a fabric or cloth is provided on the outer side of a ring-shaped core to obtain a composite of the ring-shaped core and the fabric or cloth.

Step 1 of the third aspect of the invention is carried out by first preparing the aforementioned core of the invention. Specifically, for example, a commercially available stainless steel ring (such as a stainless steel ring by Maruho Hatsujo Kogyo) may be prepared and adjusted to the desired size as necessary.

The actual core of the third aspect of the invention is the same as explained above for the annuloplasty ring of the third aspect of the invention, and the specific and preferred examples thereof are also the same.

Step 1 of the third aspect of the invention is followed by providing a fabric or cloth on the outer side (surface) of the core of the third aspect of the invention. Specifically, the fabric or cloth is wrapped around the core of the invention while being sewn to or directly covering it. The method of sewing may be any one commonly employed in the field, and for example, the sewing may be carried out by hand sewing or machine (e.g., a sewing machine).

When a second layer of the third aspect of the invention is to be provided in the annuloplasty ring of the third aspect of the invention, step 1 is carried out by providing a second layer of the third aspect of the invention on the outer side (surface) of the core of the third aspect of the invention, and further providing a fabric or cloth on its outer side (surface). In this case, the method may be, as a specific example, a method in which a second layer of the third aspect of the invention is used to cover a core of the third aspect of the invention and both ends are sealed with liquid silicone, for example, or a method in which a die that is larger than the core of the third aspect of the invention is prepared, and the core and second layer of the third aspect of the invention are inserted therein and integrally molded.

The actual second layer of the third aspect of the invention is the same as explained above for the annuloplasty ring of the third aspect of the invention, and the specific and preferred examples thereof are also the same.

Step 1 of the third aspect of the invention can produce a composite of a ring-shaped core with a fabric or cloth.

[Step 2 of third aspect of the invention]

Step 2 of the third aspect of the invention is a step of obtaining a first layer which comprises the composite obtained by step 1 of the third aspect of the invention, an impregnation layer comprising a fabric or cloth impregnated with a hydrogel, and a hydrogel layer comprising a hydrogel.

The method of contacting the composite with the hydrogel may be a method commonly employed in the relevant field, a specific example of which is the method described above for "annuloplasty ring of the third aspect of the invention" (method of spraying or coating hydrogel).

In step 2 of the third aspect of the invention, a method using a die may be employed in addition to or instead of the method described above. A method using a die may be, specifically, a method of anchoring the composite onto the central axis of the hydrogel-containing container. This method allows the hydrogel to be uniformly impregnated into the composite. Specifically, the shape of the container may be cylindrical or polygonal columnar such as triangular columnar, square columnar or pentagonal columnar. In a method using such a die, the time required to fix the hydrogel is from several seconds to 24 hours, with a fixing temperature of from 0°C to room temperature, and with the fixing being optionally followed by a time period for drying as necessary.

The actual hydrogel for step 2 of the third aspect of the invention is the same as explained above for the annuloplasty ring of the third aspect of the invention, and the specific and preferred examples thereof are also the same.

By carrying out step 2 of the third aspect of the invention it is possible to obtain a first layer which comprises an impregnation layer containing a fabric or cloth impregnated with a hydrogel, and a hydrogel layer comprising a hydrogel.

In the production method according to the third aspect of the invention, the step of adding the crosslinking agent (hereunder also abbreviated simply as "step 3 of the third aspect of the invention) may be carried out after steps 1 and 2 of the third aspect of the invention. By carrying out step 3 of the third aspect of the invention, the hydrogel itself in the annuloplasty ring of the third aspect of the invention becomes chemically crosslinked. The type of crosslinking agent and the method of crosslinking are the same as explained above for the annuloplasty ring of the third aspect of the invention, and the specific and preferred examples thereof are also the same.

<Valvuloplasty kit of third aspect of the invention>

The valvuloplasty kit of the third aspect of the invention (hereunder also referred to as "kit of the third aspect of the invention") comprises an annuloplasty ring of the third aspect of the invention, and a holder and/or sizer. The annuloplasty ring of the third aspect of the invention will be understood by referring to the valvuloplasty kit of the first aspect of the invention described above.

EXAMPLES

The present invention will now be explained in more specific detail by Examples, with the understanding that the scope of the invention is not to be limited by the Examples.

Production Examples, Examples and Comparative Examples relating to the annuloplasty ring of the first aspect of the invention will now be described.

<Production Example 1: Production of annuloplasty ring of the invention>

Using a stainless steel ring (700 micrometer diameter, by Maruho Hatsujo Kogyo) as the core material, a silicone tube (product of Silicone Techno) was used to cover the outer periphery, liquid silicone (TSE382-C, product of Momentive Performance Materials, Japan) was filled into both ends, and the mixture was allowed to stand overnight at 50°C for encapsulation. Polyethylene terephthalate yarn (33T15) was also used to wrap around the outer side a fabric (manufactured by Fukui Tateami Co., Ltd.) having a density of 38 to 39 wale/inch and 90 course/inch, and this was sewn together (this will hereunder be referred to as the "sample" for Production Example 1).

The sample was then immersed in Milli-Q and degassed for 30 minutes or longer at 50°C, ≤15 hPa. Next, the sample was slowly immersed in a 9.5% w/w gelatin solution at 50°C (beMatrix gelatin LS-H by Nitta Gelatin Inc.) and allowed to stand at 50°C. After 15 minutes, the sample was slowly removed and placed on the yarn with the seam surface facing upward (Keiryu Chosei No. 0.1, product of Toray Co., Ltd.), and allowed to stand. After 5 minutes, it was again slowly immersed in the gelatin solution, slowly removed immediately thereafter and placed on the yarn with the seam surface facing downward, and allowed to stand. After 5 minutes, it was yet again slowly immersed in the gelatin solution, slowly removed immediately thereafter and placed on the yarn with the seam surface facing upward, and allowed to stand. After 5 minutes, it was once more again slowly immersed in the gelatin solution, slowly removed immediately thereafter and placed on the yarn with the seam surface facing downward, and allowed to stand. After drying for 30 minutes at room temperature, the sample was dipped in 15 mL of a 0.2% glutaraldehyde solution (prepared from 50% glutaraldehyde solution, product of Tokyo Chemical Industry Co., Ltd.) and reacted for 3 hours at room temperature. After the reaction, the mixture was immersed in 15 mL of 60% glycerin water (product of Kenei Pharmaceutical Co., Ltd., JP pharmacopeia grade glycerin) and allowed to stand at room temperature, and after 30 minutes the immersion solution was replaced with fresh glycerin water. This procedure was carried out twice, and after the third glycerin washing, the sample was removed and dried overnight at room temperature to obtain an annuloplasty ring. Next, the obtained annuloplasty ring was placed in an aluminum bag containing a silica gel desiccant (PC (Shiro) 5G by Toyota Kako Co., Ltd.) and a deoxygenating agent (Tamotsu VX100 by OhE Chemicals Inc.), and irradiated with a 30 kilogray electron beam in the presence of dry ice (the electron beam irradiation being carried out by Shi-Atex Co., Ltd. under commission). An annuloplasty ring of the invention was thus obtained.

The other experimental conditions for the electron beam irradiation were as follows.

Irradiation device: Electron beam irradiation device (for example, Dynamitron electron accelerator by RDI)
Dosimeter: CTA dosimeter device (for example, UV1800 spectrophotometer by Shimadzu Corp.)
Dose meter: CTA dose meter (for example, FTR-125 by FujiFilm Corp.)
Acceleration voltage: 4.8 megavolt
Current: 20 milliampere
Table speed (outer periphery): 18.0 m/min
Irradiation direction: One side
Number of irradiations: 2 times
Support material: cardboard stack
Other: The absorbance before irradiation was measured with a dose meter before electron beam irradiation. The absorbance after irradiation was measured after removing the dose meter following electron beam irradiation. The radiation dose was calculated based on the difference in absorbance before and after irradiation.

<Production Example 2: Production of annuloplasty ring of the invention>

An annuloplasty ring of the invention was produced in the same manner as Production Example 1, except that the glutaraldehyde concentration was changed to 0.05%.

<Production Example 3: Production of annuloplasty ring of the invention>

An annuloplasty ring of the invention was produced in the same manner as Production Example 1, except that the glutaraldehyde concentration was changed to 1.5%.

<Production Example 4: Production of annuloplasty ring>

A fabric made of polyethylene terephthalate was wrapped around the core material of Production Example 1 to produce an annuloplasty ring.

<Production Example 5: Production of annuloplasty ring>

An annuloplasty ring similar to the annuloplasty ring of Production Example 1 (but without electron beam irradiation) was produced.

<Examples 1 to 3 and Comparative Examples 1 and 2; Hemolytic Test Examples>

[Preparation of sample for hemolytic test]

Annuloplasty rings of the invention produced by Production Examples 1 to 3 and 5 were cut open with a scalpel into 7 mm × 10 mm sheets, from the seam surface side. The cross-sections at both ends were observed with a microscope (VHX8000 by Keyence Corp.), and the film thickness of the gelatin gel including the fabric at the peak section was measured and the average value was recorded as the film thickness.
Each thickness-measured film was then placed on a 1 cm × 1 cm rubber sheet (1 t 500-square silicone rubber sheet, product of As One Corp.) with the silicone tube side facing downward, and sewn with a running stitch using ETHIBOND 5-0$^R$ (product of Johnson & Johnson), to prepare a sample for hemolytic testing. A suitable amount of a silicon adhesive (TSE392W by Momentive Performance Materials, Japan) was coated onto the back side of the sample, and anchored with an adhesive onto the bottom of a polypropylene (PP) container (14 mL No. 4 by Maruemu Corp.).

For the annuloplasty ring of Production Example 4, a sample for hemolytic testing was prepared by the same procedure as Production Examples 1 to 3 and 5, but without measurement of the film thickness. A hemolytic testing sample composed entirely of silicone rubber was also prepared based on the method described above.

[Fabrication of tube for hemolytic test]

A pump tube (silicone tube for high-strength roller pump: 3.2 × 6.4, product of As One Corp.) was cut out to a size of 45 cm. Next, a pipette (2 mL pipette, product of SUMILON) was cut to match the 1.1 mL mark, and the cut 2 mL pipette was inserted into one end of a 45 cm tube. In order to prevent clogging of blood by the pipette fragments, a metering liquid feed pump (NRP-3000 by Eyela) was used to suck out Milli-Q solution from the nozzle and discharge it from the silicone tube end. In order to detect any problems with the nozzle, Milli-Q solution was discharged from the silicone tube end and ejected from the nozzle end. Nozzle tubes found to have problems were excluded, and acceptable products were prepared by discharging water through the tube and drying the tube interior.

[Confirmation of hemolysis]

Hemolysis of commercially available blood (sodium citrate-added rabbit whole blood by Nippon SLC Corp.) was confirmed. After placing 10 mL of physiological saline (product of Otsuka Pharmaceutical Factory, Inc., 500 mL plastic bottle) in a centrifuge tube (15 mL polypropylene centrifuge tube, product of Corning, Inc.), 0.2 mL of blood was added. Centrifugal separation was carried out with a high-speed centrifuge (CF16RN by Hitachi) under conditions of 750 × g, 5 minutes. For each sample, 200 μL of supernatant was added to 3 wells of a 96-well plate (96-well plate, Violamo Corp.), and the Optical Density (OD) was measured with a plate reader (SpectraMax190, product of Molecular Devices, Japan) (wavelength: 576 nm). The OD was confirmed to be no greater than 0.014 for all of the samples, and they were therefore used for testing.

[Confirmation of flow rate]

The amount of Milli-Q solution throughput per 30 seconds and the average flow rate based on the diameter of the pipette were determined using a metering liquid feed pump and tube, and the rotational speed was set so that the average flow rate was 7 m/sec.

[Preparation of negative control blood and positive control blood]

After pouring 600 μL of physiological saline and 600 μL of blood into a 1.5 mL tube (1.5 mL of low-protein-adsorption microtube by HI-TEC Corp.), the mixture was heated at 37°C for 70 minutes (as a negative control). After then placing 600 μL of distilled water (product of Otsuka Pharmaceutical Factory, Inc., 500 mL plastic bottle) and 600 μL of blood into a 1.5 mL tube, the mixture was heated at 37°C for 70 minutes (as a positive control). The 1.5 mL tube was centrifuged with a microcentrifuge (Model3740 by Kubota) under conditions of 800 × g, 5 minutes. For the negative control, 200 μL of supernatant was added to 3 wells of a 96-well plate, and for the positive control, 180 μL of distilled water and 20 μL of supernatant were added to the 96-well plate and the mixture was diluted 10-fold (N = 3). The OD was measured with a plate reader (wavelength: 576 nm).

[Hemolytic testing]

The metering liquid feed pump was placed in a large incubator (Model 3950, product of Thermo) set to 38.5°C. After adding 5 mL of physiological saline to the 15 mL centrifuge tube, 5 mL of blood was added to prepare 2-fold diluted blood, and the mixture was warmed for 10 minutes in a water bath at 37°C. The PP container was clamped in the vertical position and the tube was set in the metering liquid feed pump with the silicone tube side of the nozzle tube in contact with the bottom of the PP container. Setting was with the long axis end of the sample in the direction of depth. The nozzle was anchored to a clamp set at a height of 3 mm in the vertical direction from the sample surface, in such a manner that the blood impacted with the impacting surface, with the spraying rate of the fluid sprayed from the nozzle tip being in a uniform range (within a region of 12 times the nozzle radius). The heated blood was transferred from the centrifuge tube in a constant amount at a low rotational speed, taking care so that air did not enter the PP container. An electric pipetter (Pipet-Aid XP by Drummond Co.) was used to transfer the remaining blood in the centrifuge tube into the PP container. Blood was discharged onto the sample for 1 hour at a rotational rate for a flow rate of 7 m/sec, based on the previously determined flow rate. Upon completion of the test, the total amount of blood was collected and stored in a 15 mL centrifuge tube. The centrifuge tube was centrifuged with a high-speed centrifuge under conditions of $750 \times g$, 5 minutes. After taking 200 $\mu$L of supernatant for each sample and adding it to 3 wells of a 96-well plate, the OD was measured with a plate reader (wavelength: 576 nm). When the absorbance of the supernatant was high, it was diluted as appropriate prior to measurement. The hemolysis rate was calculated using the following formula 3.

Hemolysis rate = (Absorbance of sample - absorbance of negative control)/(Absorbance of positive control - absorbance of negative control) - (Absorbance of sample composed of silicone rubber alone     <Formula 3> - absorbance of negative control)/(Absorbance of positive control - absorbance of negative control)

Upon completion of testing for all of the samples, the average flow rate was determined from the Milli-Q throughput per 30 seconds and the diameter of the pipette, by which it was confirmed that the average flow rate had not significantly changed from the start of the test.

[Results of hemolytic testing]

Table 1 below shows the results of hemolytic testing using the hemolytic test samples for the annuloplasty rings of Production Examples 1 to 5, as described above.
For convenience, the results for the annuloplasty rings of Production Examples 1 to 3 are shown as Examples 1 to 3, and the results for the annuloplasty rings of Production Examples 4 and 5 are used as Comparative Examples 1 and 2, respectively.

[Table 1]

|  | Example 1 (ring of Production Example 1) | Example 2 (ring of Production Example 2) | Example 3 (ring of Production Example 3) | Comp. Example 1 (ring of Production Example 4) | Comp. Example 2 (ring of Production Example 5) |
|---|---|---|---|---|---|
| Glutaraldehyde concentration (%) | 0.2 | 0.05 | 1.5 | None | 0.2 |
| Electron beam irradiation | Yes | Yes | Yes | No | No |
| Film thickness ($\mu$m) | 250 (N=46) | 258 (N=46) | 208 (N=40) | None | 250 (N=54) |
| Hemolysis rate (%) | 14 (N=4) | 15.6 (N=4) | 23.6 (N=4) | 120.5 (N=3) | 42.3 (N=4) |

As clearly seen in Table 1, when using the annuloplasty rings of Production Examples 1 to 3, i.e. the annuloplasty rings of the invention, the hemolysis rates were 14% (Example 1), 15.6% (Example 2) and 23.6% (Example 3), respectively. When using the annuloplasty rings of Production Examples 4 and 5, on the other hand, the hemolysis rates were 120.5% (Comparative Example 1) and 42.3% (Comparative Example 2), respectively. These results demonstrated that the hemolysis rate can be significantly reduced with the annuloplasty ring of the invention. Comparison between Example 1 and Comparative Example 2, in particular, surprisingly showed that the hemolysis rate is drastically improved by electron beam irradiation. When Examples 1 and 2 were compared with Example 3, it was seen that the hemolysis rate is further improved by lowering the glutaraldehyde concentration (to less than 1.5%, for example).
These results demonstrated that the annuloplasty ring of the invention is highly superior from the viewpoint of hemolysis rate.
Production Examples, Examples and Comparative Examples relating to the annuloplasty ring of the second aspect of the invention will now be described.

<Production Example 6: Production of annuloplasty ring of the invention>

Using 80 mm stainless steel wire (0.7 mm stainless steel wire, product of Hikari Mogol Co.) as the core material, the periphery was covered with a silicone tube (product of Silicone Techno), and a fabric (manufactured by Fukui Tateami Co., Ltd.) having a density of 38 to 39 wale/inch and 90 course/inch, composed of polyethylene terephthalate yarn (33T12 by Teijin, Ltd.), was wrapped around its outer side and sewn (this will hereunder be referred to as the sample for Production Example 6).
Next, a 10 mm length of a $0.7 \times 2.0$ mm silicone tube (product of Laboran) was attached to one end of the core material so that the fabric was at the center section of the core material, and then 10 mm each of a 2.0 × 4.0 mm silicone tube (product of Laboran) and a 4.0 mm × 6.0 mm silicone tube (product of Laboran) were taken and attached over the previous tube, and 80 mm of a 6.0 × 8.0 mm silicone tube (product of Laboran) was further attached over this, to prepare a die.

Into the die there was injected 1.2 mL to 1.4 mL of a 9.5% w/w gelatin solution at 50°C (beMatrix gelatin LS-H by Nitta Gelatin Inc.) together with the sample, and a silicone tube was attached to the opposite end of the die and capped. After allowing the mixture to stand for 30 to 60 minutes in a refrigerator at 4°C, the sample was removed out of the refrigerator and a scalpel was used to form two notches on the silicone tube side at spacings of 5 mm to 7 mm. The notched sections were torn, and the silicone tube was removed.

After immersing the removed sample in a 0.05% glutaraldehyde solution (prepared from 50% glutaraldehyde solution, Tokyo Chemical Industry Co., Ltd.), reaction was conducted for 3 hours at room temperature. After the reaction, the mixture was immersed in 60% glycerin water (product of Kenei Pharmaceutical Co., Ltd., JP pharmacopeia grade glycerin) and allowed to stand at room temperature, and after 30 minutes the immersion solution was replaced with fresh glycerin water. This procedure was carried out twice, and after the third washing with glycerin water, the sample was removed and dried overnight at room temperature to obtain an annuloplasty ring of the invention.

<Production Example 7: Production of annuloplasty ring of the invention>

An annuloplasty ring of the invention was produced in the same manner as Production Example 6, except that the glutaraldehyde concentration was changed to 0.5%.

<Production Example 8: Production of annuloplasty ring of the invention>

Using 80 mm stainless steel wire (0.7 mm stainless steel wire, product of Hikari Mogol Co.) as the core material, the outer periphery was covered with a silicone tube (product of Silicone Techno), and a fabric (manufactured by Fukui Tateami Co., Ltd.) having a density of 38 to 39 wale/inch and 90 course/inch, composed of polyethylene terephthalate yarn (33T12 by Teijin, Ltd.) was wrapped around its outer side and sewn (this will hereunder be referred to as the sample for Production Example 8).

Next, the sample was slowly immersed in a 9.5% w/w gelatin solution at 50°C (beMatrix gelatin LS-H by Nitta Gelatin Inc.) and allowed to stand at 50°C for at least 15 minutes. The sample was then slowly removed and allowed to stand at room temperature so as to cause the fabric section to rise up. After 5 minutes, it was again slowly immersed in the gelatin solution, slowly removed immediately thereafter, and allowed to stand at room temperature for 30 minutes or longer.

Next, a 10 mm length of a 0.7 × 2.0 mm silicone tube (product of Laboran) was attached to one end of the core material so that the fabric was at the center section of the core material, and then 10 mm each of a 2.0 × 4.0 mm silicone tube (product of Laboran) and a 4.0 mm × 6.0 mm silicone tube (product of Laboran) were taken and attached over the previous tube, and 80 mm of a 6.0 × 8.0 mm silicone tube (product of Laboran) was further attached over this, to prepare a die.

Into the die there was injected 1.2 mL to 1.4 mL of a 9.5% w/w gelatin solution at 50°C (beMatrix gelatin LS-H by Nitta Gelatin Inc.) together with the sample, and a silicone tube was attached to the opposite end of the die and capped. After allowing the mixture to stand for 30 to 60 minutes in a refrigerator at 4°C, the sample was removed out of the refrigerator and a scalpel was used to form two notches on the silicone tube side at spacings of 5 mm to 7 mm. The notched sections were torn, and the silicone tube was removed.

After immersing the removed sample in a 0.2% glutaraldehyde solution (prepared from 50% glutaraldehyde solution, Tokyo Chemical Industry Co., Ltd.), reaction was conducted for 3 hours at room temperature. After the reaction, the mixture was immersed in 60% glycerin water (product of Kenei Pharmaceutical Co., Ltd., JP pharmacopeia grade glycerin) and allowed to stand at room temperature, and after 30 minutes the immersion solution was replaced with fresh glycerin water. This procedure was carried out twice, and after the third washing with glycerin water, the sample was removed and dried overnight at room temperature to obtain an annuloplasty ring of the invention.

<Production Example 9: Production of annuloplasty ring>

A fabric made of polyethylene terephthalate was wrapped around the core material of Production Example 6 to produce an annuloplasty ring.

<Production Example 10: Production of annuloplasty ring>

A fabric made of polyethylene terephthalate was wrapped around the core material of Production Example 6, and a silicone tube was further wrapped around its outer periphery to produce an annuloplasty ring.

<Measurement of arithmetic mean roughness ratio>

The annuloplasty rings of the invention produced in Production Examples 6 to 8 and 10 were each cut into test pieces of 4 mm or longer and colored on both sides of the sample using color spray (Color Spray ZERO by Shinto-Family Co., Ltd.), and then dried. Next, a One-Shot 3D Measuring Macroscope (VR-6000 by Keyence Corp.) was used to measure the arithmetic mean roughness on the front side and back side of the annuloplasty ring of the invention in an arbitrary range of 4 mm (the detailed conditions being as follows). Specifically, the arithmetic mean roughness was measured in a range of 4 mm for each of a total of 3 locations, at both ends and the center section of the ring-shaped annuloplasty ring when stretched out in a linear manner.

The "front side", for Production Examples 6 to 8, is the front side of the fabric impregnated with the hydrogel (corresponding to the first layer of the second aspect of the invention), as the side which does not contact with the silicone tube (corresponding to the second layer of the second aspect of the invention). The "back side" is the back side of the fabric impregnated with the hydrogel (corresponding to the first layer of the second aspect of the invention), as the side that contacts with the silicone tube (corresponding to the second layer of the second aspect of the invention).

For Production Example 10, on the other hand, the front side is the surface of the silicone tube, as the side that is not in contact with the polyethylene terephthalate fabric. The "back side" is the back side of the silicone tube, as the side that contacts with the polyethylene terephthalate fabric.

The arithmetic mean roughness ratio for the front and back sides was then calculated by the following formula 4.

Arithmetic mean roughness ratio = arithmetic mean roughness of front side of first layer of second aspect of the invention/arithmetic mean roughness of back side of first layer of second aspect of the invention          <Formula 4>

<Measuring conditions>

· Roughness standard: JIS B 0601:1994, 2001
· Cutoff value: 0.8 mm
· Reference value: 4 mm

<Measurement of flexibility in direction outward from surface>

The annuloplasty rings of the invention produced in Production Examples 6 to 8 and the annuloplasty rings produced in Production Examples 9 and 10 were placed in a low-temperature thermohygrostat set to 37°C, 80% RH for 24 H or longer and allowed to swell, and the improvement in flexibility was evaluated with a small benchtop tester (EZ-SX by Shimadzu Corp.).

Specifically, each annuloplasty ring was set in a jig (product of Akimoto Koki Co.) and placed on a compression stand, the center section was pressed in the compression direction with a $\varphi$1 cm compression rod, and the load at 1.07 mm displacement was recorded (speed: 2.5 mm/min). The value of the obtained load was used to evaluate the rate of improvement in flexibility in the direction outward from the surface, for each annuloplasty ring. This measuring system measures the force (load) necessary to move (displace) the annuloplasty ring 1.07 mm, as mentioned above. This is based on the fact that movement of the annulus in the direction outward from its surface corresponds to movement (displacement) of the annulus by 1.07 mm (Reference: J Anatomy, 2020; 237:209-224). This measurement method therefore measures the load (force) at 1.07 mm displacement, by which it is possible to derive that a lower value corresponds to more excellent flexibility in the direction outward from the surface.

More specifically, the reference for the evaluation method was the value of the load of the annuloplasty ring of Production Example 9 which had the simplest construction, dividing the value of the load of each annuloplasty ring of Production Examples 6 to 8 and 10 by the value for the annuloplasty ring of Production Example 9, to evaluate the improvement rate in flexibility in the direction outward from the surface. For example, with the value of the load of the annuloplasty ring in Production Example 9 defined as 1.0, when the value of the load of the annuloplasty ring in Production Example 6 was 0.7, the value of the load of the annuloplasty ring in Production Example 6 was recorded as 70% (0.7), based on 100% as the load of the annuloplasty ring in Production Example 9 (1.0). Based on the calculation results, the improvement rate in the flexibility in the direction outward from the surface of the annuloplasty ring of Production Example 6 was evaluated to be 30% (100% - 70%). Thus, a higher value for the improvement rate in the flexibility (%) corresponds to more excellent flexibility in the direction outward from the surface.

<Measurement of gelatin solubility>

The gelatin solubilities of the annuloplasty rings produced in Production Examples 6 to 9 were each measured. Specifically, a gelatin film was prepared from each gelatin used in Production Examples 6 to 9, the film was allowed to stand for 1 hour in an aqueous solution at 37°C, and the solubility was measured by visually confirming the state.

<Summary of evaluation results>

Table 2 below shows the results for the arithmetic mean roughness ratio, flexibility in the direction outward from the surface, and gelatin solubility, for the annuloplasty rings of Production Examples 6 to 10.

For convenience, the results for the annuloplasty rings of Production Examples 6 to 8 are shown as Examples 4 to 6, and the results for the annuloplasty rings of Production Examples 9 and 10 are used as Comparative Examples 3 and 4, respectively.

[Table 2]

|  | Example 4 (ring of Production Example 6) | Example 5 (ring of Production Example 7) | Example 6 (ring of Production Example 8) | Comp. Example 3 (ring of Production Example 9) | Comp. Example 4 (ring of Production Example 10) |
|---|---|---|---|---|---|
| Glutaraldehyde concentration (%) | 0.05 | 0.5 | 0.2 | None | None |
| Load value (N) (N = 6) | 0.78 ±0.09 | 0.99 ±0.06 | 0.9 | 1.12 ±0.00 | 1.06 ±0.11 |
| Arithmetic mean roughness ratio | 0.09 | 0.04 | 0.14 | - | 1.33 |
| Flexibility improvement rate (%) (N ≥ 3) | 30% | 11% | 19% | - | 5% |
| Solubility | Good (Not dissolved) | Good (Not dissolved) | Good (Not dissolved) | - | - |

As clearly seen from Table 2, with the annuloplasty rings of Examples 6 to 8, the values (%) representing the improvement rate in flexibility in the direction outward from the surface were 30% (Example 4), 11% (Example 5) and 19% (Example 6), and the arithmetic mean roughness ratios were 0.09 (Example 4), 0.04 (Example 5) and 0.14 (Example 6), respectively.

With the annuloplasty ring of Comparative Example 4, on the other hand, the value (%) representing the improvement rate in flexibility in the direction outward from the surface was 5%, and the ratio of the arithmetic mean roughness was 1.33.

It was thus demonstrated that using an annuloplasty ring with a specific arithmetic mean roughness ratio (such as 0.04 to 0.14), as in Examples 4 to 6, improves the flexibility in the direction outward from the surface. Moreover, the fact that flexibility was exhibited, while the gelatin also did not dissolve, indicated high stability. When Examples 4 and 6 and Example 5 were compared, it was seen that the flexibility further improved if the arithmetic mean roughness ratio was 0.09 (Example 4) to 0.14 (Example 6) (Example 4: 30% and Example 6: 19%, compared to Example 5: 11%). In particular, it was found that the flexibility is maximally improved when the ratio of the arithmetic mean roughness is 0.09 (Example 4) (30%).

These results demonstrated that the annuloplasty ring of the invention is highly superior from the viewpoint of flexibility in the direction outward from the surface. If the flexibility in the direction outward from the surface of the annuloplasty ring is satisfactory, this will allow the annuloplasty ring to follow natural movement during use, thus further lowering risk of detachment between biological tissue and the annuloplasty ring. The annuloplasty ring of the invention having such flexibility can therefore provide a major contribution in the technical field.

It is conjectured that the same results obtained with the annuloplasty rings for Examples 4 to 6 can be obtained even with electron beam irradiation as in Examples 1 to 3 described above.

Production Examples, Examples and Comparative Examples relating to the annuloplasty ring of the third aspect of the invention will now be described.

<Production Example 11: Production of gelatin sheet>

A releasable film (PUREX by Teijin-DuPont Films) and a stainless steel frame (Akimoto Koki Co.) were placed in a styrol rectangular case (Model 5 by As One Corp.). A 9.5% w/w gelatin solution (beMatrix gelatin LS-H by Nitta Gelatin Inc.) and the styrol rectangular case were preheated at 65°, and 5 mL of the gelatin was added dropwise until it evenly filled the stainless steel frame. When the gelatin did not become uniformly even, it was covered and allowed to stand at 65°C until dissolved. The gelatin was then dried for 30 minutes at room temperature. The dried sample was immersed in 0.05% glutaraldehyde (product of Tokyo Chemical Industry Co., Ltd.) and reacted for 3 hours at room temperature. After the reaction, the mixture was immersed in 60% glycerin water (product of Kenei Pharmaceutical Co., Ltd., JP pharmacopeia grade glycerin) and allowed to stand at room temperature, and after 30 minutes the immersion solution was replaced with fresh glycerin water. This procedure was carried out twice, and after the third washing with glycerin water, the sample was removed and vacuum-dried to obtain a gelatin sheet.

<Production Example 12: Production of gelatin sheet>

A gelatin sheet was produced in the same manner as Production Example 11, except that the glutaraldehyde concentration was changed to 0.2%.

<Production Example 13: Production of gelatin sheet>

A gelatin sheet was produced in the same manner as Production Example 11, except that the glutaraldehyde concentration was changed to 0.3%.

<Production Example 14: Production of gelatin sheet>

A gelatin sheet was produced in the same manner as Production Example 11, except that the glutaraldehyde concentration was changed to 0.5%.

<Stress measurement>

The gelatin sheets produced in Production Examples 11 to 14 were punched out with dumbbell-shaped #8 punching teeth (for convenience, the punched gelatin sheets will also be referred to as "test pieces"). Next, the thickness was measured with a film thickness meter (VL-50 by Mitsutoyo Corp.) at 3 points: the top, center and bottom of the test piece before and after water absorption treatment, and the average value was recorded as the film thickness of the sample. Next, the stress of each gelatin sheet before and after water absorption treatment was evaluated using a small benchtop tester (EZ-SX by Shimadzu Corp.).
The water absorption treatment referred to here is treatment in which the gelatin sheet is allowed to stand for 60 minutes in ultrapure water at 37°C.
The stress referred to above is the stress (N) per unit area ($mm^2$), and the maximum stress was measured using a tensile jig (500 N screw-type flat grip by Shimadzu Corp.), sandwiching the test piece from above and below at a location 8.5 mm from the edge, and displacing the test piece until it fractured (speed: 200 mm/min).
The ratio of stress before and after water absorption treatment was calculated by the following formula 5.

Stress ratio = Stress on test piece after water absorption treatment/stress on test piece before water absorption treatment                    <Formula 5>

Separately, a silicone rubber sheet (hereunder also referred to as "Production Example 15") was prepared, and the values of the stress before and after water absorption treatment and their ratio were measured by the same procedure described above. The measured values are shown in Table 3 below.

[Table 3]

|  | Production Example 11 | Production Example 12 | Production Example 13 | Production Example 14 | Production Example 15 |
|---|---|---|---|---|---|
| Glutaraldehyde concentration | 0.05% | 0.2% | 0.3% | 0.5% | - |

(continued)

| | Production Example 11 | Production Example 12 | Production Example 13 | Production Example 14 | Production Example 15 |
|---|---|---|---|---|---|
| Stress before water absorption treatment (N/mm$^2$) | 0.57 | 0.51 | 0.41 | 0.54 | 8.63 |
| Stress after water absorption treatment (N/mm$^2$) | 0.04 | 0.2 | 0.19 | 0.33 | 7.46 |
| Stress ratio value | 0.07 | 0.4 | 0.46 | 0.6 | 0.86 |

<Evaluation of von Mises stress>

The von Mises stress was evaluated for annuloplasty rings having gelatin derived from the gelatin sheets of Production Examples 11 to 14 as a constituent element, an annuloplasty ring having the construction of Production Example 5 as a constituent element, and an annuloplasty ring having polyethylene terephthalate (PET) as a constituent element. The specific method of evaluation was as follows.

The von Mises stress represents the state of stress produced inside an object as a single value, and it is a non-directional scalar quantity which is commonly used in the field for evaluation of product performance. The von Mises stress can be determined by computer simulation, and therefore the following annuloplasty ring model was created on a computer to calculate the von Mises stress.

[Creation of annuloplasty ring model]

(1) Obtaining S-S curve

S-S curves (also referred to as "stress-strain curves") were obtained for cobalt alloy, silicone rubber, PET fabric, gelatin crosslinked with a glutaraldehyde concentration of 0.05%, gelatin crosslinked with a glutaraldehyde concentration of 0.2%, gelatin crosslinked with a glutaraldehyde concentration of 0.3% and gelatin crosslinked with a glutaraldehyde concentration of 0.5%, as constituent elements of the annuloplasty ring model of (2) below, with reference to JIS Standards (JIS L1096:2010, JIS K6251:2017, JIS Z2241).

The S-S curve is obtained to reflect the relationship between stress and strain as a mechanical property unique to each constituent element, in order to carry out the model construction of (2) below and calculate the von Mises stress via simulation.

For cobalt alloy (Poisson's ratio: 0.332, Young's modulus: 199,391), silicone rubber (Poisson's ratio: 0.499, Young's modulus: 2.660) and PET fabric (Poisson's ratio: 0.499, Young's modulus: 2.947), the obtained S-S curves were approximately linear, and these were reflected in the model construction described below, for a linear material. However, since the S-S curve was non-linear for gelatin, curve fitting was carried out using a superelastic body model (Yeoh model), and this was reflected in the model construction. The superelastic body model is a strain energy density function commonly used for analysis of superelastic bodies, and it is known that it can be precisely approximated in a wide range of strain (see Japanese Unexamined Patent Publication No. 2021-131330 and Japanese Unexamined Patent Publication No. 2012-185042, for example).

(2) Model construction

The following annuloplasty ring models 1 to 6 were prepared. The following models 1 to 4 comprise the gelatins of Production Examples 11 to 14, respectively, as constituent elements. Model 5 comprises the silicone rubber sheet of Production Example 15 as a constituent element. Model 6 has a PET fabric as a constituent element.

Each model was constructed using the commercially available software Marc Mentat 2021.1.

Model 1: Cobalt alloy (diameter: 0.7 mm) as the core of the invention, silicone rubber (diameter: 2.0 mm, thickness: 0.65 mm) as the second layer of the invention, and a PET fabric (diameter: 2.4 mm, thickness: 0.2 mm) covered with gelatin (0.05% glutaraldehyde crosslinking) (diameter: 2.84 mm, thickness: 0.22 mm) as the first layer of the invention.

Model 2: Cobalt alloy (diameter: 0.7 mm) as the core of the invention, silicone rubber (diameter: 2.0 mm, thickness: 0.65 mm) as the second layer of the invention, and a PET fabric (diameter: 2.4 mm, thickness: 0.2 mm) covered with gelatin (0.2% glutaraldehyde crosslinking) (diameter: 2.84 mm, thickness: 0.22 mm) as the first layer of the invention.

Model 3: Cobalt alloy (diameter: 0.7 mm) as the core of the invention, silicone rubber (diameter: 2.0 mm, thickness: 0.65 mm) as the second layer of the invention, and a PET fabric (diameter: 2.4 mm, thickness: 0.2 mm) covered with gelatin (0.3% glutaraldehyde crosslinking) (diameter: 2.84 mm, thickness: 0.22 mm) as the first layer of the invention.

Model 4: Cobalt alloy (diameter: 0.7 mm) as the core of the invention, silicone rubber (diameter: 2.0 mm, thickness: 0.65 mm) as the second layer of the invention, and a PET fabric (diameter: 2.4 mm, thickness: 0.2 mm) covered with gelatin (0.5% glutaraldehyde crosslinking) (diameter: 2.84 mm, thickness: 0.22 mm) as the first layer of the invention.

Model 5: Cobalt alloy (diameter: 0.7 mm) as the core of the invention, silicone rubber (diameter: 2.0 mm, thickness: 0.65 mm) as the second layer of the invention, and a PET fabric (diameter: 2.4 mm, thickness: 0.2 mm) covered with silicon rubber (diameter: 2.84 mm, thickness: 0.22 mm) as the first layer of the invention.

Model 6: Cobalt alloy (diameter: 0.7 mm) as the core of the invention, silicone rubber (diameter: 2.0 mm, thickness: 0.65 mm) as the second layer of the invention, and a PET fabric (diameter: 2.4 mm, thickness: 0.2 mm) as the first layer of the invention.

[Calculation of von Mises stress]

(1) Anchoring jig

The anchoring jig was treated as a rigid body, ignoring deformation of the anchoring jig during deformation of the annuloplasty ring. The anchoring jig is a container used for the measurement described below, being a cubic container with dimensions of 8 mm × 8 mm × 8 mm.

(2) Calculation conditions

Fig. 4 shows a schematic diagram of an analytical model for calculation of the von Mises stress for models 1 to 6 (Fig. 4 shows the annuloplasty ring 1, X-axial direction 7 and anchoring jig 8). For this analytical model, the von Mises stress was evaluated when the anchoring part fixed by the anchoring jig was moved 2.55 mm in the X-axial direction (the commercially available software Marc Mentat 2021.1 was used for calculation of the von Mises stress). In order to reduce the computer load for calculation, a 1/2 symmetrical model equivalent to the schematic diagram of Fig. 4 was used. Since the anchoring jig was treated as a rigid body (ignoring deformation of the anchoring jig), the displacement of the anchoring jig was imposed on the contact surface of the annuloplasty ring, rather than modeling the anchoring jig.

For this measurement, the von Mises stress was calculated when 2.55 mm movement was carried out in the X-axial direction. This is based on the fact that movement of the annulus in the X-axial direction (i.e. within the surface) corresponds to movement (displacement) of the annulus by 1.7 mm (Reference: The Journal of Thoracic and Cardiovascular Surgery, Volume 122, Number 4 666-673). Therefore, the von Mises stress for 1.7 mm displacement is calculated as described above, with a lower value indicating lower force on the annuloplasty ring produced by movement within the surface, which consequently lowers the risk of damage to the annuloplasty ring. In actual calculation, the value of 2.55 mm is used, as the value of 1.7 mm multiplied by a safety factor of 1.5.

The term "within the surface" refers to the direction of the arrow 3 in Fig. 3, and the opposite term "direction outward from its surface" means the direction of the arrow 2 in Fig. 3.

[Calculation results]

Table 4 shows the von Mises stress (maximum) calculated as described above. The location where the von Mises stress is maximum is at the anchoring part boundary, regardless of whether gelatin is present, and it is thought to be affected by switching of the boundary conditions. The von Mises stress was higher at the center (of the plane of symmetry of the analytical model), excluding the anchoring part boundary.

[Table 4]

| | Model 1 (Production Example 11) | Model 2 (Production Example 12) | Model 3 (Production Example 13) | Model 4 (Production Example 14) | Model 5 (Production Example 15) | Model 6 |
|---|---|---|---|---|---|---|
| Glutaraldehyde concentration | 0.05% | 0.2% | 0.3% | 0.5% | - | - |
| Stress before water absorption treatment (N/mm$^2$) | 0.57 | 0.51 | 0.41 | 0.54 | 8.63 | - |
| Stress after water absorption treatment (N/mm$^2$) | 0.04 | 0.2 | 0.19 | 0.33 | 7.46 | - |

(continued)

|  | Model 1 (Production Example 11) | Model 2 (Production Example 12) | Model 3 (Production Example 13) | Model 4 (Production Example 14) | Model 5 (Production Example 15) | Model 6 |
|---|---|---|---|---|---|---|
| Stress ratio value | 0.07 | 0.4 | 0.46 | 0.6 | 0.86 | - |
| von Mises stress maximum (Mpa) | 607 | 615 | 619 | 630 | 660 | 693 |

[Supplementary notes]

The von Mises stress is calculated on a computer in terms of its properties, as mentioned above. Since this calculation is generally carried out in the field the results are reasonably reliable, but the calculation results may still be verified from a different perspective. The following method may be used for verification, without being particularly limitative.

(1) Prior preparation

As an example, annuloplasty rings are prepared which are the same as the annuloplasty ring of model 6 and the annuloplasty rings of models 1 to 4. The method may be carried out with reference to the description in the present specification. Marks are then formed on both the left and right at a position 8 mm from the tip of the ring. Separately, cable covers (CA-KK22 by Sanwa) are cut to 1.2 cm. The cut cable covers are lined up at 10.5 mm spacings, and epoxy resin putty is filled into the cable covers. Next, each annuloplasty ring is placed in the epoxy resin putty at a position 8 mm from the tip and allowed to stand overnight until hardened.

(2) Measurement

A One-Shot 3D (VR-6000 by Keyence Corp.) was used to measure the diameter at the marked location 8 mm from the tip of the annuloplasty ring.

(3) Tensile test

The sample was displaced (at a speed of 2.5 mm/min, for example) with a small benchtop tester (EZ-SX by Shimadzu Corp.) for the specified amount of yield strain. After displacement, the diameter at a position 8 mm from the tip was again measured with a One-Shot 3D. The deformation of each annuloplasty ring before and after displacement was calculated. The specified value for the yield strain may be set at or above the value of the yield strain measured when calculating the von Mises stress.

Next, the degree of deformation is calculated by comparing the degree of deformation of each of the annuloplasty rings of models 1 to 4 and the degree of deformation of the annuloplasty ring of model 6. The calculation results are compared with the degree of deformation estimated from the measurement results for yield strain, and it is confirmed whether or not they tend to match. The aforementioned verification was carried out for the Examples and they tended to match, thus confirming the validity of the calculation system for von Mises stress.

<Evaluating number of fatigue fractures>

The von Mises stress calculated as described above was inserted into the SN curve for Elgiloy commonly used in the field (http://www.hightechalloys.eu/pdf/Elgiloy_Flyer.pdf), and the number of fatigue fractures in each model was calculated as shown in Table 5.

The SN curve is a curve representing the relationship between the stress repeatedly applied at a constant amplitude, and the number of load cycles until fracture, as the fatigue fracture of the material. The number of fatigue fractures is the number of times required until the sample fractures. Consequently, a larger number of fatigue fractures means that a corresponding greater number of times is required until the annuloplasty ring fractures, and it may therefore be considered to be stronger.

[Table 5]

| | Model 1 (Production Example 11) | Model 2 (Production Example 12) | Model 3 (Production Example 13) | Model 4 (Production Example 14) | Model 5 (Production Example 15) | Model 6 |
|---|---|---|---|---|---|---|
| Glutaraldehyde concentration | 0.05% | 0.2% | 0.3% | 0.5% | - | - |
| Stress before water absorption treatment (N/mm$^2$) | 0.57 | 0.51 | 0.41 | 0.54 | 8.63 | - |
| Stress after water absorption treatment (N/mm$^2$) | 0.04 | 0.2 | 0.19 | 0.33 | 7.46 | - |
| Stress ratio value | 0.07 | 0.4 | 0.46 | 0.6 | 0.86 | - |
| von Mises stress maximum (Mpa) | 607 | 615 | 619 | 630 | 660 | 693 |
| Number of fatigue fractures | $>1.0 \times 10^7$ | $>1.0 \times 10^7$ | $>1.0 \times 10^7$ | $>1.0 \times 10^7$ | $7.2 \times 10^6$ | $4.9 \times 10^6$ |

<Summary of evaluation results>

As clearly seen in Tables 4 and 5, using the annuloplasty rings of models 1 to 4, i.e. annuloplasty rings wherein the stress ratio value was 0.07 to 0.6 and gelatins of Production Examples 11 to 14 were used as constituent elements, resulted in markedly lower von Mises stress compared to models 5 and 6. In other words, it was found that using the annuloplasty rings of models 1 to 4 can reduce stress on the annuloplasty rings resulting from their movement within the surface (that is, it is possible to reduce concentration of stress), thereby reducing the risk of damage to the annuloplasty rings. Comparison between models 1 to 3 (Production Examples 11 to 13) and model 4 (Production Example 14) shows that the von Mises stress was lower in models 1 to 3 (Production Examples 11 to 13). In particular, since the von Mises stress was markedly lower with model 1 (Production Example 11) compared to the other models, it was found to be most superior from the viewpoint of reducing damage risk.

As clearly seen in Table 5, using the annuloplasty rings of models 1 to 4, i.e. annuloplasty rings wherein the stress ratio value was 0.07 to 0.6 and gelatins of Production Examples 1 to 4 were used as constituent elements, resulted in a marked increase in the number of fatigue fractures compared to models 5 and 6. In other words, the annuloplasty rings with gelatins of models 1 to 4 (Production Examples 11 to 14) as constituent elements had markedly low risk of damage even with repeated use.

It is conjectured that the same results obtained with the annuloplasty rings for models 1 to 4 can be obtained even with electron beam irradiation as in Examples 1 to 3 described above.

INDUSTRIAL APPLICABILITY

The annuloplasty ring of the invention (for example, the annuloplasty ring of the first aspect of the invention) has a superior effect of inhibiting hemolysis and increased biocompatibility compared to conventional products, and can reduce the risk of reoperation due to hemolysis occurring after surgery. It can therefore be utilized industrially for production of annuloplasty rings.

**Claims**

1. An annuloplasty ring comprising:

   (1) a ring-shaped core and
   (2) a first layer that comprises a fabric or cloth impregnated with a hydrogel, covering the surface of the core,

   wherein the hydrogel is crosslinked by radiation.

2. The annuloplasty ring according to claim 1, wherein (3) a second layer comprising rubber is present between the core and the first layer, and the value of the ratio between the arithmetic mean roughness of the front side of the first layer which is not in contact with the core or the second layer, and the arithmetic mean roughness of the back side of the first layer which is in contact with the core or the second layer, is 0.01 to 0.40.

3. The annuloplasty ring according to claim 1, wherein the first layer comprises an impregnation layer containing a fabric or cloth impregnated with a hydrogel, and a hydrogel layer composed of a hydrogel, the value of the ratio of the stress before subjecting the hydrogel in the hydrogel layer to water absorption treatment and the stress after subjecting the hydrogel to water absorption treatment is 0.05 to 0.80, the water absorption treatment being treatment in which the hydrogel layer is allowed to stand for 30 to 90 minutes in a solution at 30°C to 50°C.

4. The annuloplasty ring according to any one of claims 1 to 3, wherein the hydrogel is gelatin or collagen.

5. The annuloplasty ring according to claim 1, wherein the irradiation in radiation crosslinking is at least one type selected from the group consisting of X-ray irradiation, γ-ray irradiation, electron beam irradiation, UV irradiation and plasma irradiation.

6. The annuloplasty ring according to claim 5, wherein the irradiation in radiation crosslinking is electron beam irradiation.

7. The annuloplasty ring according to claim 1, wherein the fabric or cloth comprises polyester fibers.

8. The annuloplasty ring according to claim 1, wherein the hydrogel is further chemically crosslinked.

9. An annuloplasty ring comprising:

   (1) a ring-shaped core, and
   (2) a first layer comprising a fabric or cloth impregnated with a hydrogel, covering the surface of the core.

10. The annuloplasty ring according to claim 1, which is used to help prevent hemolysis in valvuloplasty.

11. A valvuloplasty kit comprising an annuloplasty ring according to claim 1, and a holder and/or sizer.

12. A method for producing an annuloplasty ring comprising (1) a ring-shaped core and (2) a first layer comprising a fabric or cloth impregnated with a hydrogel, covering the surface of the core, the method comprising a step of irradiating the annuloplasty ring with radiation.

13. The method according to claim 12, wherein the step of irradiating with radiation is a step of irradiating with one or more selected from the group consisting of X-rays, γ-rays, an electron beam, UV and plasma.

14. The method according to claim 13, wherein the step of irradiating with radiation is a step of irradiating with an electron beam.

15. The method according to claim 12, which further comprises a step of adding a crosslinking agent.

# Fig. 1

# Fig. 2

# Fig. 3

Fig. 4

Fig. 5

## INTERNATIONAL SEARCH REPORT

| International application No. |
|---|
| **PCT/JP2024/018763** |

**A. CLASSIFICATION OF SUBJECT MATTER**

*A61F 2/24*(2006.01)i; *A61L 27/04*(2006.01)i; *A61L 27/18*(2006.01)i; *A61L 27/22*(2006.01)i; *A61L 27/24*(2006.01)i; *A61L 27/28*(2006.01)i; *A61L 27/34*(2006.01)i; *A61L 27/40*(2006.01)i; *A61L 27/52*(2006.01)i
FI: A61F2/24; A61L27/52; A61L27/28; A61L27/40; A61L27/24; A61L27/22; A61L27/18; A61L27/04; A61L27/34

According to International Patent Classification (IPC) or to both national classification and IPC

**B. FIELDS SEARCHED**

Minimum documentation searched (classification system followed by classification symbols)

A61F2/24; A61L27/04; A61L27/18; A61L27/22; A61L27/24; A61L27/28; A61L27/34; A61L27/40; A61L27/52

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | US 2002/0129820 A1 (MEDTRONIC, INC.) 19 September 2002 (2002-09-19) paragraphs [0050]-[0053], [0060], [0072], fig. 1-2, 12-14 | 1, 4-8, 10-15 |
| A | | 2-3 |
| X | US 6585767 B1 (AGION TECHNOOGIES, INC.) 01 July 2003 (2003-07-01) column 6, line 61 to column 7, line 27, fig. 5-5A | 9 |
| Y | | 1, 4-8, 10-15 |
| Y | WO 2022/266119 A1 (EDWARDS LIFESCIENCES CORPORATION) 22 December 2022 (2022-12-22) paragraph [0119] | 1, 4-8, 10-15 |
| Y | CN 108904877 A (INST METAL RESEARCH CAS) 30 November 2018 (2018-11-30) paragraphs [0015], [0033], [0041], [0059] | 1, 4-8, 10-15 |

☐ Further documents are listed in the continuation of Box C.     ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **03 July 2024** | **16 July 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2024/018763**

| Patent document cited in search report | | | Publication date (day/month/year) | Patent family member(s) | | | Publication date (day/month/year) |
|---|---|---|---|---|---|---|---|
| US | 2002/0129820 | A1 | 19 September 2002 | JP | 2004-528885 | A | |
| | | | | EP | 1370202 | A2 | |
| US | 6585767 | B1 | 01 July 2003 | WO | 2000/030567 | A2 | |
| WO | 2022/266119 | A1 | 22 December 2022 | EP | 4355267 | A1 | |
| CN | 108904877 | A | 30 November 2018 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018519895 A
- JP 4503318 A
- US 6805711 B
- JP 6310167 B
- JP 2018519986 A
- US 11554015 B
- US 20230125047 A
- JP 2020157516 A
- JP 2021146242 A
- JP 2021131330 A
- JP 2012185042 A

**Non-patent literature cited in the description**

- *Jpn. J. Cardiovasc. Surg.*, 2008, vol. 37, 151-154
- *Journal of Heart Valve Disease*, 2005
- *J Anatomy*, 2020, vol. 237, 209-224
- *The Journal of Thoracic and Cardiovascular Surgery*, vol. 122 (4), 666-673